# EUROPEAN PATENT SPECIFICATION

(11) **EP 1 471 890 B1**
(45) Date of publication and mention of the grant of the patent: **27.09.2006**
(21) Application number: 03710927.9
(22) Date of filing: 06.02.2003
(51) Int. Cl.: A61K 9/34

(54) **PHARMACEUTICAL DOSAGE FORM FOR MUCOSAL DELIVERY**
PHARMAZEUTISCHE DARREICHUNGSFORM ZUR MUKOSALEN VERABREICHUNG
FORME PHARMACEUTIQUE DESTINEE A ETRE ADMINISTREE PAR VOIE MUCOSALE

(30) Priority: 07.02.2002 US 355703 P
(43) Date of publication of application: 03.11.2004
(73) Proprietor: Pharmacia Corporation, St. Louis, MO 63141 (US)
(72) Inventor: MARTINO, Alice, C., Kalamazoo, MI 49009 (US); PIERMAN, Steven, A., Portage, MI 49002 (US); NOACK, Robert, M., Ann Arbor MI 48103 (US); BRITTEN, Nancy, Portage, MI 49024 (US)
(74) Representative: Wood, David John
(86) International application number: PCT/US2003/003836
(87) International publication number: WO 2003/066029

(56) References cited:
- EP-A- 0 181 966
- WO-A-95/20377
- WO-A-99/22769

## Description

### FIELD OF THE INVENTION

The present invention relates to pharmaceutical compositions suitable for intraoral administration to provide delivery of a drug via the oral mucosa.

### BACKGROUND OF THE INVENTION

Pharmaceutical dosage forms suitable for placement in the mouth of a subject, for example in the sublingual or buccal spaces of the mouth, to permit absorption of a drug into the subject's bloodstream via the oral mucosa, are well known. See for example Rathbone, ed. (1996) *Oral Mucosal Drug Delivery,* Marcel Dekker; in particular the articles therein by Kellaway & Warren, "Mucoadhesive hydrogels for buccal delivery," pp. 221-239, and by Rathbone *et al.,* "Systemic oral mucosal drug delivery and delivery systems," pp. 241-284.

It is often desired that such intraoral dosage forms, particularly those intended for sublingual administration, release the drug rapidly to provide onset of therapeutic benefit as soon as possible after administration. For this reason dosage forms for intraoral administration are conveniently formulated as "soft" tablets, *i.e.,* tablets subjected to only a low degree of compaction during manufacture and/or having a relatively low amount of binding agent, to enable rapid disintegration in the oral cavity and thereby rapid drug release. Typically such tablets are not coated, as commonly used film coatings can delay disintegration of a tablet and result in slower drug release than may be desirable.

The low compaction of typical intraoral, particularly sublingual, tablets and the lack of a protective coating thereon tends to result in such tablets being friable and therefore subject to breakage and attrition during packaging, shipping and dispensing.

U.S. Patent No. 6,326,028 to Nivaggioli *et al.* discloses a tablet coating comprising gellan gum. Such a coating is said to be useful for tablets to be taken orally, and to confer benefits in appearance, identification, mouth feel, reduced dust, stability, color and/or swallowability.

International Patent Publication No. WO 00/40226 discloses compounds useful in treating sexual dysfunction in men and women, these compounds being of formula (I) 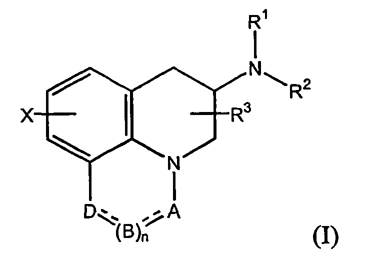 or pharmaceutically acceptable salts thereof, wherein
- R¹, R² and R³: are the same or different and are H, C₁₋₆ alkyl (optionally phenyl substituted), C₃₋₅ alkenyl or alkynyl or C₃₋₁₀ cycloalkyl, or where R³ is as above and R¹ and R² are cyclized with the attached N atom to form pyrrolidinyl, piperidinyl, morpholinyl, 4-methylpiperazinyl or imidazolyl groups;
- X: is H, F, Cl, Br, I, OH, C₁₋₆ alkyl or alkoxy, CN, carboxamide, carboxyl or (C₁₋₆ alkyl)carbonyl;
- A: is CH, CH₂, CHF, CHCl, CHBr, CHI, CHCH₃, C=O, C=S, CSCH₃, C=NH, CNH₂, CNHCH₃, CNHCOOCH₃, CNHCN, SO₂ or N;
- B: is CH, CH₂, CHF, CHCl, CHBr, CHI, C=O, N, NH or NCH₃, and n is 0 or 1; and
- D: is CH, CH₂, CHF, CHCl, CHBr, CHI, C=O, O, N, NH or NCH₃;
with various provisos indicated therein. WO 00/40226 further contemplates prescription of the drug (*R*)-5,6-dihydro-5-(methylamino)-4H-imidazo[4,5-*ij*]-quinolin-2(1H)-one (*Z*)-2-butenedioate (1:1) to male and female subjects at a dose of 1-3 mg, to be taken 0.5-1 h before engaging in sexual activity, and indicates that at such a dose and timing of administration the drug is therapeutically effective. No information is provided as to the route of administration or nature of dosage form.

The class of compounds proposed for treatment of sexual dysfunction in WO 00/40226 was earlier disclosed in U.S. Patent No. 5,273,975 to Moon *et al.* to have therapeutically useful central nervous system activity. Certain compounds of the above class are the subject of a paper by Heier *et al.* (1997), "Synthesis and biological activities of (*R*)-5,6-dihydro-N,N-dimethyl-4H-imidazo[4,5,1-*ij*]quinolin-5-amine and its metabolites", *J. Med. Chem.* 40, 639-646.

International Patent Publication No. WO 99/16442 discloses a sustained-release tablet formulation of (*R*)-5,6-dihydro-5-(methylamino)-4H-imidazo[4,5-*ij*]-quinolin-2(1H)-one (Z)-2-butenedioate (1:1) for treatment of Parkinson's disease.

European Patent Application No. 0 992 240 discloses cGMP-PDE inhibitory compounds said to be useful in treatment of male erectile dysfunction and proposes transmucomembranous administration, for example in the form of sublingual preparations, of such compounds.

Heaton (1996), "Buccal apomorphine", *Journal of Urology* 155, 49, reports efficacy of a sublingual formulation of apomorphine in treatment of male non-organic erectile dysfunction.

U.S. Patent No. 5,985,889 to El-Rashidy *et al.* proposes sublingual administration of apomorphine for treatment of male psychogenic erectile dysfunction. Various sublingual tablet formulations of apomorphine hydrochloride are disclosed therein.

International Patent Publication No. WO 00/35457 proposes use of apomorphine for treatment of male organic, e.g., vasculogenic, erectile dysfunction, and exemplifies use of a sublingual tablet formulation of apomorphine hydrochloride. WO 00/35457 further suggests that nausea, a common side effect of apomorphine, can be controlled by inclusion of an anti-emetic agent such as nicotine in the formulation.

U.S. Patent No. 6,121,276 to El-Rashidy & Ronsen discloses flavored sublingual tablets containing apomorphine hydrochloride and nicotine.

U.S. Patent No. 5,994,363 to El-Rashidy & Ronsen discloses a treatment regime with apomorphine that is said to reduce side effects such as nausea, vomiting, yawning and cardiovascular effects.

U.S. Patent Nos. 5,624,677 and 5,888,534, both to El-Rashidy *et al.* discloses a prolonged release sublingual formulation of apomorphine.

International Patent Publication No. WO 01/49292 discloses sublingual tablets of apomorphine providing prolonged release of the drug, said to be useful in treatment of Parkinson's disease.

International Patent Publication No. WO 00/4299 discloses a dosage unit comprising a water-soluble hydrocolloid and sildenafil citrate in a mucoadhesive film said to be suitable for application to the oral mucosa. Pharmacokinetic data presented in WO 00/42992 indicate no faster absorption into the bloodstream with sublingual application of such a film than with a commercial tablet formulation of sildenafil citrate (Viagra®) at the same dosage.

International Patent Publication No. WO 01/10406 discloses compositions said to be suitable for a wide range of routes of administration of sildenafil citrate, including buccal and sublingual routes. Preferred compositions disclosed are said to comprise a solution, gel, semisolid, suspension, metered dose device, transdermal patch or film. It is indicated that such compositions can include a gelling system, for example gellan gum 0.5% to 10%.

International Patent Publication No. WO 02/05820 discloses film dosage forms comprising sildenafil citrate. These dosage forms are prepared by mixing a solid dispersion of sildenafil citrate and a water soluble sugar with a hydrocolloid and optionally other ingredients, and are said, upon placement on a mucosal surface, to form a coating that subsequently disintegrates and dissolves to release sildenafil. Gellan sodium salt is listed among hydrocolloids said to be useful in such film dosage forms.

U.S. Patent No. 6,291,506 to Levin discloses that the ophthalmic drug carvedilol can be formulated for ocular administration by suspending it in an agent such as gellan gum that will increase corneal contact time with the drug. Other possible delivery modes for the drug are contemplated therein. A claim is included to a method wherein the drug is delivered by a selection of routes including sublingually.

U.S. Patent No. 6,297,240 to Embleton discloses an intraorally deliverable composition comprising an ophthalmic drug, for example a drug useful in lowering intraocular pressure.

### SUMMARY OF THE INVENTION

There is now provided a pharmaceutical tablet comprising an intraorally disintegratable core and an excipient coating adherent thereto, wherein the coating comprises gellan gum.

The tablet is suitable for intraoral administration, for example for delivery of a drug contained in the core of the tablet to a subject, for example a human subject, at least in part by absorption of the drug via oral mucosa of the subject. The term "intraoral" herein refers to administration by placement of the tablet in the mouth of the subject, where the tablet disintegrates and/or dissolves. Intraoral administration herein is therefore distinct from conventional oral administration of a tablet, wherein the tablet is swallowed prior to substantial disintegration or dissolution. For intraoral administration, the tablet can be placed in or on any part of the mouth, but placement of the tablet in the sublingual or buccal spaces is preferred.

An "intraorally disintegratable" core herein is a core that, in the absence of a coating, disintegrates readily in the mouth. Where the core comprises a drug, the drug is released and becomes available for mucosal absorption as the core disintegrates. Typically, an intraorally disintegratable core is of low hardness (*e*.*g*., less than about 4 SCU).

An "excipient coating" herein is a coating consisting, at least at the time of application of the coating to the core, only of excipient materials, *i.e*., having substantially no drug present therein. It will be understood that during manufacture and storage some migration of a drug substance can potentially occur from the core to the coating of a tablet of the invention, but this is generally minimal and does not remove such a tablet from the scope of the present invention. It is important to note that according to the invention a drug substance, if present in the tablet, is largely confined to the core where it is not commingled with gellan gum.

Therefore a further embodiment of the invention is a pharmaceutical tablet comprising a drug in a therapeutically and/or prophylactically effective amount, the tablet having an intraorally disintegratable core and a coating adherent thereto, wherein the coating comprises gellan gum, and substantially all, for example at least about 90%, of the drug is located in the core and is not commingled with gellan gum.

The present invention provides a solution to a long-standing problem in the art in that a soft tablet suitable for intraoral administration, which normally is very friable, and therefore vulnerable to breakage and attrition during manufacture, packaging, shipping and dispensing, can be made more robust without significant reduction in rate of disintegration in the mouth. A coating comprising gellan gum as contemplated herein confers such robustness, as measurable by reduced breakage and/or attrition of the tablet prior to administration, yet does not result in appreciable retardation of disintegration upon placement of the tablet in the mouth. Tablet coatings widely used for swallowable tablets, for example film coatings comprising a cellulosic polymer such as hydroxypropylmethylcellulose or ethylcellulose, are generally unsuitable for tablets intended for intraoral administration because these coatings tend to inhibit intraoral disintegration of such tablets and/or mucosal absorption of a drug contained in such tablets. Another problem with film coatings comprising a cellulosic polymer is that such coatings tend to become detached from the underlying tablet core as film flakes in the mouth. This can lead to an unpleasant oral sensation and can induce the subject to swallow the tablet rather than retain it in the mouth.

Furthermore, the highly friable tablets that are generally used for intraoral drug delivery are difficult to coat with cellulosic polymers by processes known in the art. By contrast, a coating comprising gellan gum as contemplated herein can be applied to a highly friable core according to a coating process disclosed herein, without unacceptable breakage or attrition of cores during the coating process.

Tablets of the invention can possess one or more additional advantages over intraorally administrable tablets of prior art. For example, a tablet of the invention can have one or more of a glossy and/or color-enhancing appearance, improved organoleptic quality such as flavor and/or mouth feel, and improved mucoadhesion resulting in better retention or "seating" of the tablet at the site of placement and/or enhanced mucosal absorption of a drug contained in the tablet.

Coated tablets as provided herein are generally less expensive and/or more convenient to prepare, package and dispense than other hydrocolloid-containing dosage forms such as films and gels.

Other features, advantages and benefits of the invention will be apparent from the description that follows.

### DETAILED DESCRIPTION OF THE INVENTION

A tablet of the invention can be a placebo tablet, *i.e.,* containing no drug or other active agent in the core thereof. Preferably a tablet of the invention contains in the core a therapeutically and/or prophylactically useful amount of a drug, more preferably a drug that is advantageously delivered by intraoral administration. In principle any drug is deliverable intraorally, but in practice intraoral administration is particularly advantageous for certain classes of drugs and drug products, for example:
(a) drugs more readily or more rapidly absorbed via the oral mucosa than in the gastrointestinal tract;
(b) drugs subject to first-pass metabolism in the liver;
(c) smoking cessation products, for example those containing nicotine;
(d) antibacterial drugs;
(e) drugs to treat ophthalmic disorders;
(f) analgesics, antipyretics and anti-inflammatories, for example NSAIDs (nonsteroidal anti-inflammatory drugs) including selective cyclooxygenase-2-(COX-2) inhibitory drugs;
(g) drugs to treat sexual dysfunction, for example dopaminergic agonists; *etc.*

"First-pass metabolism" as mentioned above is a problem with gastrointestinal delivery of some drugs, it being noted that absorption of a drug into the bloodstream from the gastrointestinal tract exposes the drug to metabolism in the liver during its first pass through the circulatory system. By contrast, blood from capillary beds in the oral mucosa drains directly into systemic circulation and avoids first-pass metabolism. See Rathbone *et al., op. cit.*

More generally, the drug present in the core of a tablet of the invention can be selected from the following illustrative classes: ACE inhibitors; α-adrenergic agonists; β-adrenergic agonists; α-adrenergic blockers; β-adrenergic blockers (beta blockers); alcohol deterrents; aldose reductase inhibitors; aldosterone antagonists; amino acids; anabolics; analgesics (both narcotic and non-narcotic); anesthetics; anorexics; antacids; anthelmintics; antiacne agents; antiallergics; antiandrogens; antianginal agents; antianxiety agents; antiarrythmics; antiasthmatics; antibacterial agents and antibiotics; antialopecia and antibaldness agents; antiamebics; antibodies; anticholinergic drugs; anticoagulants and blood thinners; anticolitis drugs; anticonvulsants; anticystitis drugs; antidepressants; antidiabetic agents; antidiarrheals; antidiuretics; antidotes; antiemetics; antiestrogens; antiflatulents; antifungal agents; antigens; antiglaucoma agents; antihistaminics; antihyperactives; antihyperlipoproteinemics; antihypertensives; antihyperthyroid agents; antihypotensives; antihypothyroid agents; anti-infectives; anti inflammatories (both steroidal and nonsteroidal); antimalarial agents; antimigraine agents; antineoplastics; antiobesity agents; antiparkinsonian agents and antidyskinetics; antipneumonia agents; antiprotozoal agents; antipruritics; antipsoriatics; antipsychotics; antipyretics; antirheumatics; antisecretory agents; anti-shock medications; antispasmodics; antithrombotics; antitumor agents; antitussives; antiulceratives; antiviral agents; anxiolytics; bactericidins; bone densifiers; bronchodilators; calcium channel blockers; carbonic anhydrase inhibitors; cardiotonics and heart stimulants; chemotherapeutics; choleretics; cholinergics; chronic fatigue syndrome medications; CNS stimulants; coagulants; contraceptives; cystic fibrosis medications; decongestants; diuretics; dopamine receptor agonists; dopamine receptor antagonists; enzymes; estrogens; expectorants; gastric hyperactivity medications; glucocorticoids; hemostatics; HMG CoA reductase inhibitors; hormones; hypnotics; immunomodulators; immunosuppressants; laxatives; medicaments for oral and periodontal diseases; miotics; monoamine oxidase inhibitors; mucolytics; multiple sclerosis medications; muscle relaxants; mydriatics; narcotic antagonists; NMDA receptor antagonists; oligonucleotides; ophthalmic drugs; oxytocics; peptides, polypeptides and proteins; polysaccharides; progestogens; prostaglandins; protease inhibitors; respiratory stimulants; sedatives; serotonin uptake inhibitors; sex hormones including androgens; smoking cessation drugs; smooth muscle relaxants; smooth muscle stimulants; thrombolytics; tranquilizers; urinary acidifiers; urinary incontinence medications; vasodilators; vasoprotectants; and combinations thereof

It will be understood that any reference herein to a particular drug compound includes tautomers, stereoisomers, enantiomers, salts and prodrugs of that compound and is not specific to any one solid-state form of the drug.

In one embodiment a drug contained in the core of the tablet is a smoking cessation drug, for example nicotine, a nicotine metabolite or a non-nicotine aid to smoking cessation such as bupropion or ibogaine.

Illustratively, a smoking cessation drug can be selected from nicotine and metabolites thereof (*e.g.,* cotinine, norcotinine, nornicotine, nicotine N-oxide, cotinine N-oxide, 3-hydroxycotinine and 5-hydroxycotinine), ibogaine, bupropion and metabolites thereof (*e:g.*, the erythro- and threo-amino alcohols of bupropion, the erythro-amino diol of bupropion and hydroxybupropion), lobeline, selegiline, risperidone and its 9-hydroxy metabolite, desmethylselegiline, substituted pyridine derivatives (*e.g*., 1-[(6-chloro-3-pyridinyl)methyl]-2-imidazolidine, 1-[(6-chloro-3-pyridinyl)methyl]-2-imidazothiazole and analogs thereof), methcamylamine, desipramine, fluoxetine, ropinirole, trimethaphan, trimethaphan camsylate, doxepin, 2-(3-chlorophenyl)-3,5,5-trimethyl-2-morpholinol, anxiolytics (*e.g*., isovaleramide), γ-vinyl GABA (GVG), epibatidine and derivatives thereof, 7-azabicyclo-[2.2.1]-heptane and -heptene compounds, naltrexone, nalmefene, ketamine, hexamethonium, pentolinium, dihydro-β-erythroidine, erysodine, d-tubocurarine, pempidine, chlorisondamine, amantadine, hetero-oxy alkanamines, benzylidene- and cinnamylidene-anabasines, azaindole-ethylamine derivatives, N-(pyridinylmethyl)-heterocyclylideneamines and NK-1 receptor antagonists (*e.g*., 9-bromo-1,2,3,4,5,6-hexahydro-1,5-methano-pyrido[1,2-a][1,5]diazocin-8-one).

In another embodiment a drug contained in the core of the tablet is an antibacterial drug. Illustratively such a drug can be an antibiotic, for example an aminoglycoside, amphenicol, ansamycin, carbapenem, cephalosporin, cephamycin, monobactam, oxacephem, penicillin, lincosamide, macrolide, polypeptide or tetracycline; or a synthetic antibacterial, for example a 2,4-diaminopyrimidine, nitrofuran, oxazolidinone, quinolone or analog thereof, sulfonamide or sulfone. Presently preferred antibacterials include the following illustrative examples: amikacin, azithromycin, cefixime, cefoperazone, cefotaxime, ceftazidime, ceftizoxime, ceftriaxone, chloramphenicol, ciprofloxacin, clindamycin, colistin, domeclocycline, doxycycline, erythromycin, gentamicin, lincomycin, linezolid, mafenide, methacycline, minocycline, neomycin, norfloxacin, ofloxacin, oxytetracycline, pirlirnycin, polymyxin B, pyrimethamine, silver sulfadiazine, sulfacetamide, sulfisoxazole, tetracycline, tobramycin, trimethoprim and combinations thereof. In one embodiment an antibacterial drug present in the core of the tablet is an oxazolidinone, for example selected from (*S*)-N-[[3-[3-fluoro-4-[4-(hydroxyacetyl)-1-piperazinyl]phenyl]-2-oxo-5-oxazolidinyl]methyl]acetamide (eperezolid), (*S*)-N-[[3-[3-fluoro-4-[4-(morpholinyl)phenyl]-2-oxo-5-oxazolidinyl]methyl]acetamide (linezolid), N-[(5*S*)-3-[3-fluoro-4-[4-(2-fluoroethyl)-3-oxo-1-piperazinyl]phenyl-2-oxo-5-oxazolidinyl]methyl]acetamide, (*S*)-N-[[3-[5-(3-pyridyl)thiophen-2-yl]-2-oxo-5-oxazolidinyl]methyl]acetamide, and (*S*)-N-[[3-[5-(4-pyridyl)pyrid-2-yl]-2-oxo-5-oxazolidinyl]methyl]acetamide hydrochloride.

In another embodiment a drug contained in the core of the tablet is an antimigraine agent. Illustratively such an agent can be an alkylxanthine, for example caffeine; a dopamine D₂ receptor agonist, for example alpiropride or lisuride; a GABA_{A} receptor modulator, for example ganaxolone; a 5-hydroxytriptamine (5-HT) receptor agonist, for example almotriptan, eletriptan, frovatriptan, naratriptan, rizatriptan, sumatriptan or zolmitriptan; ergot or a derivative thereof, for example ergotamine or dihydroergotamine; or a vasomodulator, for example dotarizine, fonazine or lomerizine.

In another embodiment a drug contained in the core of the tablet is useful in treating or preventing an ophthalmic disorder.

Illustratively such an ophthalmic drug can be an antibacterial, for example selected from the classes listed above.

Alternatively or in addition, such an ophthalmic drug can illustratively be an antiglaucoma or intraocular pressure lowering agent, such as (a) an α-adrenergic agonist or sympathomimetic, *e.g*., adrenolone, apraclonidine, brimonidine or dipivefrin; (b) a β-adrenergic blocker, *e.g.*, acebutolol, adaprolol, alprenolol, atenolol, betaxolol, bufetolol, bufuralol, bunitrolol, bunolol, bupranolol, carteolol, carvedilol, cetamolol, dexpropanolol, labetalol, levobunolol, metipranolol, metoprolol, nadolol, nifenalol, oxyprenolol, penbutolol, pindolol, practolol, pronethalol, propranolol, sotalol, timolol, tolamolol, toliprolol or vaninolol; (c) a carbonic anhydrase inhibitor, *e.g*., acetazolamide or dorzolamide; or (d) a prostaglandin or analog thereof, *e.g*., PGF_{2α} analogs such as bimatoprost, latanoprost, travoprost and unoprostone isopropyl.

Alternatively or in addition, such an ophthalmic drug can illustratively be a miotic, *e.g.,* carbachol, physostigmine or pilocarpine.

Alternatively or in addition, such an ophthalmic drug can illustratively be an anti-inflammatory agent, for example an NSAID, more preferably a selective COX-2 inhibitory drug, for example selected from those listed below.

In another embodiment a drug contained in the core of the tablet is an analgesic, antipyretic or anti-inflammatory agent, *e.g*., aceclofenac, acemetacin, *e*-acetamidocaproic acid, acetaminophen, acetaminosalol, acetanilide, acetylsalicylic acid (aspirin), *S*-adenosylmethionine, alclofenac, alclometasone, alfentanil, algestone, allylprodine, alminoprofen, aloxiprin, alphaprodine, aluminum bis(acetylsalicylate), amcinonide, amfenac, aminochlorthenoxazin, 3-amino-4-hydroxybutyric acid, 2-amino-4-picoline, aminopropylon, aminopyrine, amixetrine, ammonium salicylate, ampiroxicam, amtolmetin guacil, anileridine, antipyrine, antrafenine, apazone, beclomethasone, bendazac, benorylate, benoxaprofen, benzpiperylon, benzydamine, benzylmorphine, bermoprofen, betamethasone, bezitramide, α-bisabolol, bromfenac, *p*-bromoacetanilide, 5-bromosalicylic acid acetate, bromosaligenin, bucetin, bucloxic acid, bucolome, budesonide, bufexamac, bumadizon, buprenorphine, butacetin, butibufen, butophanol, carbamazepine, carbiphene, carprofen, carsalam, celecoxib, chlorobutanol, chloroprednisone, chlorthenoxazin, choline salicylate, cinchophen, cinmetacin, ciramadol, clidanac, clobetasol, clocortolone, clometacin, clonitazene, clonixin, clopirac, cloprednol, clove, codeine, codeine methyl bromide, codeine phosphate, codeine sulfate, cortisone, cortivazol, cropropamide, crotethamide, deflazacort, desomorphine, desonide, desoximetasone, dexamethasone, dexoxadrol, dextromoramide, dezocine, diampromide, diclofenac, difenamizole, difenpiramide, diflorasone, diflucortolone, diflunisal, difluprednate, dihydrocodeine, dihydrocodeinone enol acetate, dihydromorphine, dihydroxyaluminum acetylsalicylate, dimenoxadol, dimepheptanol, dimethylthiambutene, dioxaphetyl butyrate, dipipanone, diprocetyl, dipyrone, ditazol, droxicam, emorfazone, enfenamic acid, enoxolone, epirizole, eptazocine, etersalate, ethenzamide, ethoheptazine, ethoxazene, ethylmethylthiambutene, ethylmorphine, etodolac, etofenamate, etonitazene, etoricoxib, eugenol, felbinac, fenbufen, fenclozic acid, fendosal, fenoprofen, fentanyl, fentiazac, fepradinol, feprazone, floctafenine, fluazacort, flucloronide, flufenamic acid, flumethasone, flunisolide, flunixin, flunoxaprofen, fluocinolone acetonide, fluocinonide, fluocortin butyl, fluocortolone, fluoresone, fluorometholone, fluperolone, flupirtine, fluprednidene, fluprednisolone, fluproquazone, flurandrenolide, flurbiprofen, formocortal, fosfosal, gentisic acid, glafenine, glucametacin, glycol salicylate, guaiazulene, halcinonide, halometasone, haloprednone, hydrocodone, hydrocortamate, hydrocortisone, hydromorphone, hydroxypethidine, ibufenac, ibuprofen, ibuproxam, imidazole salicylate, indomethacin, indoprofen, isofezolac, isoladol, isomethadone, isonixin, isoxepac, isoxicam, ketobemidone, ketoprofen, ketorolac, *p*-lactophenetide, lefetamine, levorphanol, lofentanil, lonazolac, lomoxicam, loxoprofen, lysine acetylsalicylate, mazipredone, meclofenamic acid, medrysone, mefenamic acid, meperidine, meprednisone, meptazinol, mesalamine, metazocine, methadone, methotrimeprazine, methylprednisolone, metiazinic acid, metofoline, metopon, mofebutazone, mofezolac, morazone, morphine, morphine hydrochloride, morphine sulfate, morpholine salicylate, myrophine, nabumetone, nalbuphine, 1-naphthyl salicylate, naproxen, narceine, nefopam, nicomorphine, nifenazone, niflumic acid, nimesulide, 5'-nitro-2'-propoxyacetanilide, norlevorphanol, normethadone, nonnorphine, norpipanone, olsalazine, opium, oxaceprol, oxametacine, oxaprozin, oxycodone, oxymorphone, oxyphenbutazone, papaveretum, paramethasone, paranyline, parecoxib, parsalmide, pentazocine, perisoxal, phenacetin, phenadoxone, phenazocine, phenazopyridine hydrochloride, phenocoll, phenoperidine, phenopyrazone, phenyl acetylsalicylate, phenylbutazone, phenyl salicylate, phenyramidol, piketoprofen, piminodine, pipebuzone, piperylone, piprofen, pirazolac, piritramide, piroxicam, pranoprofen, prednicarbate, prednisolone, prednisone, prednival, prednylidene, proglumetacin, proheptazine, promedol, propacetamol, propiram, propoxyphene, propyphenazone, proquazone, protizinic acid, proxazole, ramifenazone, remifentanil, rimazolium metilsulfate, rofecoxib, salacetamide, salicin, salicylamide, salicylamide *o*-acetic acid, salicylic acid, salicylsulfuric acid, salsalate, salverine, simetride, sufentanil, sulfasalazine, sulindac, superoxide dismutase, suprofen, suxibuzone, talniflumate, tenidap, tenoxicam, terofenamate, tetrandrine, thiazolinobutazone, tiaprofenic acid, tiaramide, tilidine, tinoridine, tixocortol, tolfenamic acid, tolmetin, tramadol, triamcinolone, tropesin, valdecoxib, viminol, xenbucin, ximoprofen, zaltoprofen or zomepirac.

In a particular embodiment such a drug is a selective COX-2 inhibitory drug, for example a compound of formula (II): 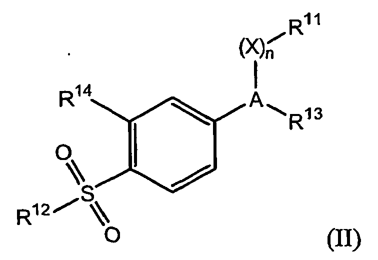 or a prodrug thereof or a pharmaceutically acceptable salt thereof, wherein:
- A: is a substituent selected from partially unsaturated or unsaturated heterocyclyl and partially unsaturated or unsaturated carbocyclic rings, preferably a heterocyclyl group selected from pyrazolyl, furanonyl, isoxazolyl, pyridinyl, cyclopentenonyl and pyridazinonyl groups;
- X: is O, S or CH₂;
- n: is 0 or 1;
- R¹¹: is at least one substituent selected from heterocyclyl, cycloalkyl, cycloalkenyl and aryl, and is optionally substituted at a substitutable position with one or more radicals selected from alkyl, haloalkyl, cyano, carboxyl, alkoxycarbonyl, hydroxyl, hydroxyalkyl, haloalkoxy, amino, alkylamino, arylamino, nitro, alkoxyalkyl, alkylsulfinyl, halo, alkoxy and alkylthio;
- R¹²: is methyl, amino or aminocarbonylalkyl;
- R¹³: is one or more radicals selected from hydrido, halo, alkyl, alkenyl, alkynyl, oxo, cyano, carboxyl, cyanoalkyl, heterocyclyloxy, alkyloxy, alkylthio, alkylcarbonyl, cycloalkyl, aryl, haloalkyl, heterocyclyl, cycloalkenyl, aralkyl, heterocyclylalkyl, acyl, alkylthioalkyl, hydroxyalkyl, alkoxycarbonyl, arylcarbonyl, aralkylcasbonyl, aralkenyl, alkoxyalkyl, arylthioalkyl, aryloxyalkyl, aralkylthioalkyl, aralkoxyalkyl, alkoxyaralkoxyalkyl, alkoxycarbonylalkyl, aminocarbonyl, aminocarbonylalkyl, alkylaminocarbonyl, N-arylaminocarbonyl, N-alkyl-N-arylaminocarbonyl, alkylaminocarbonylalkyl, carboxyalkyl, alkylamino, N-arylamino, N-aralkylamino, N-alkyl-N-aralkylamino, N-alkyl-N-arylamino, aminoalkyl, alkylaminoalkyl, N-arylaminoalkyl, N-aralkylaminoalkyl, N-alkyl-N-aralkylaminoalkyl, N-alkyl-N-arylaminoalkyl, aryloxy, aralkoxy, arylthio, aralkylthio, alkylsulfinyl, alkylsulfonyl, aminosulfonyl, alkylaminosulfonyl, N-arylaminosulfonyl, arylsulfonyl and N-alkyl-N-arylaminosulfonyl, R¹³ being optionally substituted at a substitutable position with one or more radicals selected from alkyl, haloalkyl, cyano, carboxyl, alkoxycarbonyl, hydroxyl, hydroxyalkyl, haloalkoxy, amino, alkylamino, arylamino, nitro, alkoxyalkyl, alkylsulfinyl, halo, alkoxy and alkylthio; and
- R¹⁴: is selected from hydrido and halo.

In a preferred composition according to the present embodiment the selective COX-2 inhibitory drug is a compound having the formula (III): 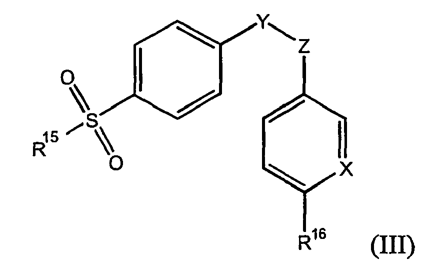 where R¹⁵ is a methyl, amino or imide group, R¹⁶ is hydrogen or a C₁₋₄ alkyl or alkoxy group, X is N or CR¹⁷ where R¹⁷ is hydrogen or halogen, and Y and Z are independently carbon or nitrogen atoms defining adjacent atoms of a five- to six-membered ring that is unsubstituted or substituted at one or more positions with oxo, halo, methyl or halomethyl groups. Preferred such five- to six-membered rings are cyclopentenone, furanone, methylpyrazole, isoxazole and pyridine rings substituted at no more than one position.

In another preferred composition according to the present embodiment the selective COX-2 inhibitory drug is a compound having the formula (IV): 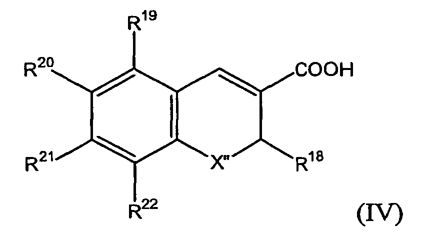 or a prodrug thereof or a pharmaceutically acceptable salt thereof, where X" is O, S or N-lower alkyl; R¹⁸ is lower haloalkyl; R¹⁹ is hydrogen or halogen; R²⁰ is hydrogen, halogen, lower alkyl, lower alkoxy or haloalkoxy, lower aralkylcarbonyl, lower dialkylaminosulfonyl, lower alkylaminosulfonyl, lower aralkylaminosulfonyl, lower heteroaralkylaminosulfonyl, or 5- or 6- membered nitrogen-containing heterocyclosulfonyl; and R²¹ and R²² are independently hydrogen, halogen, lower alkyl, lower alkoxy, or aryl.

A particularly useful compound of formula (IV) is (S)-6,8-dichloro-2-(trifluoromethyl)-2H-1-benzopyran-3-carboxylic acid.

In yet another preferred composition according to the present embodiment the selective COX-2 inhibitory drug is a 5-alkyl-2-arylaminophenylacetic acid or derivative thereof Particularly useful compounds of this class are 5-methyl-2-(2'-chloro-6'-fluoroanilino)phenylacetic acid and pharmaceutically acceptable salts thereof

Illustratively, celecoxib, deracoxib, valdecoxib, parecoxib, rofecoxib, etoricoxib, 2-(3,5-difluorophenyl)-3-[4-(methylsulfonyl)phenyl]-2-cyclopenten-1-one, (*S*)-6,8-dichloro-2-(trifluoromethyl)-2H-1-benzopyran-3-carboxylic acid, 2-(3,4-difluorophenyl)-4-(3-hydroxy 3-methyl-1-butyoxy)-5-[4-(methylsulfonyl)phenyl]-3-(2H)-pyridazinone and salts thereof are useful in compositions of the present embodiment.

For example, the selective COX-2 inhibitory drug or prodrug thereof can be selected from celecoxib, valdecoxib, parecoxib, rofecoxib, etoricoxib, (*S*)-6,8-dichloro-2-(trifluoromethyl)-2H-1-benzopyran-3-carboxylic acid and salts thereof

In another embodiment, a drug contained in the core of the tablet is useful in treatment and/or prevention of sexual dysfunction in male and/or female subjects. Such a drug can illustratively be (a) a phosphodiesterase type 5 (PDE5) inhibitor, *e.g*., sildenafil, tadalafil or vardenafil, (b) a cyclic GMP phosphodiesterase inhibitor, (c) a cyclic AMP activator, (d) an α-adrenergic antagonist, *e.g*., phentolamine or yohimbine, or (e) a dopaminergic agonist, *e.g*., apomorphine. Such a drug can be a compound of formula (I) below. Alternatively, a drug contained in the core of the tablet can be other than a drug useful in treatment and/or prevention of sexual dysfunction. As another alternative, a drug contained in the core of the tablet can be useful in treatment and/or prevention of sexual dysfunction but is other than a compound of formula (I) below.

In illustrative compositions a drug useful in treatment and/or prevention of sexual dysfunction is present in the core of the tablet in an amount of about 0.05 mg to about 10 mg per tablet and is a compound of formula (I) 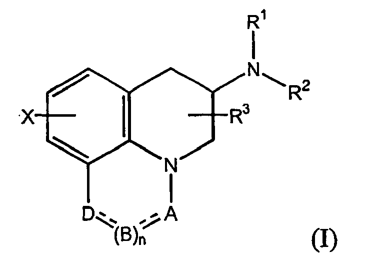 or a pharmaceutically acceptable salt thereof, wherein
- R¹, R² and R³: are the same or different and are H, C₁₋₆ alkyl (optionally phenyl substituted), C₃₋₅ alkenyl or alkynyl or C₃₋₁₀ cycloalkyl, or where R³ is as above and R¹ and R² are cyclized with the attached N atom to form pyrrolidinyl, piperidinyl, morpholinyl, 4-methylpiperazinyl or imidazolyl groups;
- X: is H, F, Cl, Br, I, OH, C₁₋₆ alkyl or alkoxy, CN, carboxamide, carboxyl or (C₁₋₆ alkyl)carbonyl;
- A: is CH, CH₂, CHF, CHCl, CHBr, CHI, CHCH₃, C=O, C=S, CSCH₃, C=NH, CNH₂, CNHCH₃, CNHCOOCH₃, CNHCN, SO₂ or N;
- B: is CH, CH₂, CHF, CHCl, CHBr, CHI, C=O, N, NH or NCH₃, and n is 0 or 1; and
- D: is CH, CH₂, CHF, CHCl, CHBr, CHI, C=O, O, N, NH or NCH₃.
It is preferred that the compound of formula (I) or salt thereof is water-soluble.

Pharmaceutically acceptable salts of a compound of formula (I) include without restriction salts of the following acids: hydrochloric, hydrobromic, sulfuric, methanesulfonic, phosphoric, nitric, benzoic, citric, tartaric, fumaric and maleic acids, and mono- and dicarboxylic acids of formula CH₃-(CH₂)ₙ-COOH and HOOC-(CH₂)ₙ₋COOH where n is 0 to 4, for example malonic acid.

Particularly preferred salts are the hydrochloride salt and the maleate, *i.e*., (*Z*)-2-butenedioate, salt.

Compounds of formula (I) and their salts can be prepared by processes known *per se,* including processes described in patent literature cited herein. However, the present invention is not restricted by the process used to prepare the therapeutic agent.

Preferred compounds of formula (I) are those disclosed generically or specifically in above-cited U.S. Patent No. 5,273,975. Especially preferred compounds are those of formula (V) 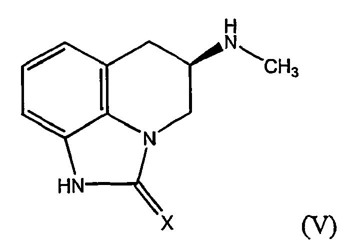 wherein X is O or S, and pharmaceutically acceptable salts thereof.

A "therapeutically effective amount" of a compound of formula (I) herein is an amount sufficient to improve sexual desire, interest or performance in a subject having a sexual dysfunction condition. A "sexual-stimulatorily effective amount" herein is an amount sufficient to improve sexual desire, interest or performance in a subject whether or not the subject has a sexual dysfunction condition. It is preferred that the amount of the compound of formula (I) or salt thereof be lower than an amount causing significant side-effects; in general it will be found that dosage amounts lower than about 5 mg, especially lower than about 3 mg, are relatively free of such side-effects.

Compounds of formula (I), in particular compounds of formula (V) and salts thereof, when formulated as described herein, can be effective at surprisingly low doses. At such low doses, despite the high aqueous solubility of compounds of formula (V) and in particular of their salts, there is generally no pronounced taste associated with the therapeutic agent. Even if a taste is detectable, it is relatively easily masked or balanced by excipients and encapsulation is normally not required.

Tablets of the invention containing a drug of formula (I) are adapted for discreet self-administration. By "discreet self-administration" herein is meant self administration shortly prior to sexual activity in a way that does not draw attention of a sexual partner to, or emphasize, the existence of a sexual dysfunction, a need for therapy or a need or desire for enhancement of sexual performance. The combination of discreetness and rapid onset that is permitted by the present invention provides a benefit in spontaneity; by contrast, prior art compositions for treating sexual dysfunction can be seriously compromised in their effectiveness if their self-administration requires premeditation and/or cannot be done discreetly, such self-administration being thereby not conducive to spontaneity. In particular, the present invention does not involve self-injection, and does not require water or other drink as an aid to swallowing.

A tablet of the invention wherein the therapeutic agent is sumanirole, (*R*)-5,6-dihydro-5-(methylamino)-4H-imidazo[4,5-*ij*]-quinolin-2(1H)-one, preferably contains about 0.05 to about 5 mg, more preferably about 0.1 to about 5 mg, still more preferably about 0.2 to about 5 mg, even more preferably about 0.5 to about 5 mg, of sumanirole free base equivalent, as free base or as salt. In one embodiment the tablet contains about 0.25 to about 3 mg, for example about 1 to about 3 mg, of sumanirole free base equivalent, as free base or as salt. If desired, the sumanirole can be only partially neutralized with acid so that free base coexists with salt in the tablet.

A tablet of the invention wherein the therapeutic agent is (*R*)-5,6-dihydro-5-(methylamino)-4H-imidazo[4,5-*ij*]-quinoline-2(1H)-thione preferably contains about 0.05 to about 5 mg, more preferably about 0.1 to about 3 mg, and most preferably about 0.25 to about 2 mg, of (*R*)-5,6-dihydro-5-(methylamino)-4H-imidazo[4,5-*ij*]-quinoline-2(1H)-thione free base equivalent, as free base or as salt. In one embodiment the tablet contains about 0.1 to about 3 mg, for example about 0.25 to about 1 mg, of (*R*)-5,6-dihydro-5-(methylamino)-4H-imidazo[4,5-*ij*]-quinoline-2(1H)-thione free base equivalent, as free base or as salt. If desired, the (*R*)-5,6-dihydro-5-(methylamino)4H-imidazo [4,5-*ij*] -quinoline-2(1H)-thione can be only partially neutralized with acid so that free base coexists with salt in the tablet.

In one embodiment a compound of formula (I) is present in a tablet of the invention in a therapeutically or sexual-stimulatorily effective amount of less than 1 mg, for example about 0.05 mg to about 0.75 mg. Surprisingly a tablet of the invention having such a low amount of the active agent can exhibit a desired degree of efficacy; further, any unpleasant taste resulting from intraoral interaction by the tablet is minimized or absent.

Tablet cores useful according to the invention can be prepared by any suitable process known in the art. Such cores are then coated with a coating composition comprising gellan gum, as more fully described below. The coating is typically present in an amount representing a weight gain of about 0.1% to about 5%, but greater or lesser amounts can be used if desired. Preferably the gellan gum constitutes about 25% to 100%, more preferably about 50% to 100%, by weight of the coating.

Any gellan gum can be used in the coating composition, but it is preferred to use a deacylated gellan gum such as that sold under the trademark Kelcogel^{™}. Optionally one or more additional gums and/or biopolymers, for example alginates, can be present in the coating composition.

The coating composition comprises a sprayable vehicle, preferably water, having dissolved or dispersed therein a gellan gum and optionally one or more additional excipients. Preferably the coating composition has a total solids concentration of about 1% to about 10% by weight, and a gellan gum concentration of about 1% to about 5% by weight.

Additional excipients present in the coating composition can include one or more buffering agents, typically at a concentration of about 0.03% to about 3% by weight; one or more plasticizers, typically at a concentration of about 0.03% to about 3% by weight; and/or one or more dispersing and/or emulsifying agents, typically at a concentration of about 0.03% to about 3% by weight. An example of a suitable buffering agent is sodium citrate. An example of a suitable plasticizer is propylene glycol. An example of a suitable dispersing or emulsifying agent is lecithin. Flavoring and coloring agents can also be included in the coating composition if desired.

The coating composition can be prepared by any suitable process involving dissolving the gellan gum and other, optional, excipients in the vehicle, preferably water. Order of addition is not critical. The water is preferably heated, for example to a temperature of about 55°C to about 85°C. Gellan gum and other excipients, if present, are added with stirring until all ingredients are homogeneously dispersed. The resulting coating liquid is preferably maintained at an elevated temperature during the stirring and subsequent spraying procedure.

Tablet cores to be coated are placed in a suitable coating apparatus, for example a coating pan, and are preferably preheated to a bed temperature of about 50°C to about 70°C. The coating liquid is sprayed on to the tablets under conditions that will be readily optimized by one of skill in the art. Spraying is continued until an amount of coating solution equivalent to a weight gain of about 0.1% to about 5% has been applied. The resulting coated tablets are preferably cooled to ambient temperature, or about 20°C to about 35°C, prior to discharge from the coating pan.

An illustrative sublingual tablet of the invention containing as active agent a salt, *e.g.,* the maleate salt, of sumanirole or (*R*)-5,6-dihydro-5-(methylamino)-4H-imidazo[4,5-*ij*]-quinoline-2(1H)-thione has a core having the following composition:

| | |
|---|---|
| active agent | 0.1-3% free base equivalent |
| mannitol | 50-90% |
| powdered sorbitol | 10-40% |
| hydroxypropylcellulose | 0-10% |
| xanthan gum | 0-5% |
| flavoring agent | 0-0.5% |
| coloring agent | 0-0.5% |
| colloidal silicon dioxide | 0-1% |
| magnesium stearate | 0.5-5% |

all percentages being by weight.

Another illustrative sublingual tablet of the invention containing as active agent a salt, *e.g*., the maleate salt, of sumanirole or (*R*)-5,6-dihydro-5-(methylamino)-4H-imidazo[4,5-*ij*]-quinoline-2(1H)-thione has a core having the following composition:

| | |
|---|---|
| active agent | 0.1-3% free base equivalent |
| lactose monohydrate | 50-85% |
| pregelatinized starch | 10-45% |
| xanthan gum | 0-5% |
| flavoring agent | 0-0.5% |
| coloring agent | 0-0.5% |
| colloidal silicon dioxide | 0-1% |
| magnesium stearate | 0.5-5% |

all percentages being by weight.

Yet another illustrative sublingual tablet of the invention containing as active agent a salt, *e.g*., the maleate salt, of sumanirole or (*R*)-5,6-dihydro-5-(methylamino)-4H-imidazo[4,5-*ij*]-quinoline-2(1H)-thione has a core having the following composition:

| | |
|---|---|
| active agent | 0.1-3% free base equivalent |
| microcrystalline cellulose | 30-70% |
| pregelatinized starch | 25-65% |
| croscarmellose sodium | 0-10% |
| xanthan gum | 0-5% |
| flavoring agent | 0-0.5% |
| coloring agent | 0-0.5% |
| colloidal silicon dioxide | 0-1% |
| magnesium stearate | 0.5-5% |

all percentages being by weight.

### EXAMPLES

The following examples illustrate aspects of the present invention but should not be construed as limitations. In these examples "compound Z" refers to (*R*)-5,6-dihydro-5-(methylamino)-4H-imidazo[4,5-*ij*]-quinoline-2(1H)-thione, maleate salt. All percentages are by weight unless otherwise indicated.

### Example 1

A sublingual tablet formulation was prepared having the following composition:

| | |
|---|---|
| compound Z | 1.11% |
| Avicel^{™} PH-101 (microcrystalline cellulose) | 46.71% |
| Starch 1500 of Colorcon (pregelatinized starch) | 44.00% |
| croscarmellose sodium NF | 5.00% |
| colloidal silicon dioxide NF | 0.50% |
| cinnamon flavor | 0.14% |
| mint flavor | 0.04% |
| color (cherry shade #1632, Crompton & Knowles) | 0.50% |
| magnesium stearate | 2.00% |

Pregelatinized starch and color were blended in a high-shear mixer for 2 minutes or until homogeneously mixed. The following ingredients were then individually layered over the resulting mixture in the high-shear mixer: compound Z; microcrystalline cellulose; colloidal silicon dioxide; croscarmellose sodium. Mixing in the high-shear mixer was resumed for a further 2 minutes. If the color was not adequately dispersed throughout the resulting mixture, mixing continued in 1 minute increments until good dispersion of color was observed. A small portion of the mixture was then removed and hand-mixed with magnesium stearate to form a magnesium stearate premix. This premix, together with the flavors, was added to the high-shear mixer and mixed for 1 minute to form a lubricated tablet stock.

The lubricated tablet stock was discharged from the high-shear mixer and stored in desiccated hermetically sealed containers until ready for tableting. Tablets were prepared by compression using 12/32 inch (approximately 9 mm) Plain/Plain tooling with slight curvature to the following specifications:

| | |
|---|---|
| tablet weight | 180 mg |
| hardness | 3-4 SCU |
| friability | <0.5% |

### Example 2

Sublingual tablets prepared as in Example 1 were coated with a gellan gum coating according to the following procedure.

A coating liquid having the following composition was prepared:

| | |
|---|---|
| gellan gum (Kelcogel^{™}) | 2.00% |
| sodium citrate | 0.13% |
| propylene glycol | 0.40% |
| lecithin | 0.20% |
| deionized water | 97.27% |

Deionized water was heated to 70°C. The other ingredients were added with stirring until all ingredients were homogeneously dispersed. The resulting coating liquid having a solids content of 2.73% was maintained at a temperature of 70°C during the stirring and subsequent spraying procedure.

Tablets of Example 1, in an amount of 700 g, were placed in a 12 inch (approximately 300 mm) coating pan and preheated to a bed temperature of 60°C. The coating liquid was sprayed on to the tablets under the following conditions:

| | |
|---|---|
| outlet air temperature | 50-60°C |
| pan speed | 16 rpm |
| air flow | 30-35 cfm (0.84-0.98 m³/minute) |
| atomizing air pressure | 10 psi (69 kPa) |
| peristaltic pump setting | 15-20 g/minute |

Spraying was continued until an amount of coating solution equivalent to a weight gain of 1.2% had been applied. The resulting coated tablets were cooled to 30°C prior to discharge from the coating pan.

### Example 3

A sublingual tablet formulation was prepared having the following composition:

| | |
|---|---|
| compound Z | 1.05% |
| mannitol, granular | 70.00% |
| sorbitol | 16.57% |
| hydroxypropylcellulose, type LH-11 | 7.00% |
| xanthan gum | 2.50% |
| colloidal silicon dioxide NF | 0.50% |
| cinnamon flavor | 0.14% |
| mint flavor | 0.04% |
| color (cherry shade #1632, Crompton & Knowles) | 0.20% |
| magnesium stearate | 2.00% |

Mannitol and color were blended in a high-shear mixer for 2 minutes or until homogeneously mixed. The following ingredients were then individually layered over the resulting mixture in the high-shear mixer: compound Z; sorbitol; hydroxypropylcellulose; xanthan gum; colloidal silicon dioxide. Mixing in the high-shear mixer was resumed for a further 2 minutes. If the color was not adequately dispersed throughout the resulting mixture, mixing continued in 1 minute increments until good dispersion of color was observed. A small portion of the mixture was then removed and hand-mixed with magnesium stearate to form a magnesium stearate premix. This premix, together with the flavors, was added to the high-shear mixer and mixed for 1 minute to form a lubricated tablet stock.

The lubricated tablet stock was discharged from the high-shear mixer and stored in desiccated hermetically sealed containers until ready for tableting. Tablets were prepared by compression using 12/32 inch (approximately 9 mm) Plain/Plain tooling with slight curvature to the following specifications:

| | |
|---|---|
| tablet weight | 190 mg |
| hardness | 3-4 SCU |
| friability | <0.5% |

### Example 4

Sublingual tablets prepared as in Example 3 were coated with a gellan gum coating according to the following procedure.

A coating liquid having the following composition was prepared:

| | |
|---|---|
| gellan gum (Kelcogel^{™}) | 2.00% |
| sodium citrate | 0.13% |
| propylene glycol | 0.40% |
| lecithin (Lipoid^{™} LS-100) | 0.20% |
| flavor | 0.30% |
| deionized water | 96.97% |

Deionized water was heated to 70°C. The other ingredients were added with stirring until all ingredients were homogeneously dispersed. The resulting coating liquid having a solids content of 3.03% was maintained at a temperature of 70°C during the stirring and subsequent spraying procedure.

Tablets of Example 1, in an amount of 700 g, were placed in a 12 inch (approximately 300 mm) coating pan and preheated to a bed temperature of 60°C. The coating liquid was sprayed on to the tablets under the following conditions:

| | |
|---|---|
| outlet air temperature | 50-60°C |
| pan speed | 16 rpm |
| air flow | 30-35 cfm (0.84-0.98 m³/minute) |
| atomizing air pressure | 10 psi (69 kPa) |
| peristaltic pump setting | 15-20 g/minute |

Spraying was continued until an amount of coating solution equivalent to a weight gain of 1.36% had been applied. The resulting coated tablets were cooled to 30°C prior to discharge from the coating pan.

### Example 5

A sublingual tablet formulation was prepared having the following composition:

| | |
|---|---|
| compound Z | 0.43% |
| Avicel^{™} PH-101 (microcrystalline cellulose) | 47.39% |
| Starch 1500 of Colorcon (pregelatinized starch) | 44.00% |
| croscarmellose sodium NF | 5.00% |
| colloidal silicon dioxide NF | 0.50% |
| cinnamon flavor | 0.14% |
| mint flavor | 0.04% |
| color (cherry shade #1632, Crompton & Knowles) | 0.50% |
| magnesium stearate | 2.00% |

Pregelatinized starch and color were blended in a high-shear mixer for 2 minutes or until homogeneously mixed. The following ingredients were then individually layered over the resulting mixture in the high-shear mixer: compound Z; microcrystalline cellulose; colloidal silicon dioxide; croscarmellose sodium. Mixing in the high-shear mixer was resumed for a further 2 minutes. If the color was not adequately dispersed throughout the resulting mixture, mixing continued in 1 minute increments until good dispersion of color was observed. A small portion of the mixture was then removed and hand-mixed with magnesium stearate to form a magnesium stearate premix. This premix, together with the flavors, was hand screened through a #20 mesh pharmaceutical screen, then added to the high-shear mixer and mixed for 1 minute to form a lubricated tablet stock.

The lubricated tablet stock was discharged from the high-shear mixer and stored in desiccated hermetically sealed containers until ready for tableting. Tablets were prepared by compression using 12/32 inch (approximately 9 mm) Plain/Plain tooling with slight curvature to the following specifications:

| | |
|---|---|
| tablet weight | 180 mg |
| hardness | 3.5-4 SCU |
| friability | <0.8% |

### Example 6

Sublingual tablets prepared as in Example 5 were coated with a gellan gum coating according to the following procedure.

A coating liquid having the following composition was prepared:

| | |
|---|---|
| gellan gum (Kelcogel^{™}) | 2.00% |
| sodium citrate | 0.13% |
| propylene glycol | 0.40% |
| lecithin (Lipoid^{™} LS-100) | 0.20% |
| hot cinnamon flavor | 0.30% |
| deionized water | 96.97% |

Deionized water was heated to 70°C. The other ingredients were added with stirring until all ingredients were homogeneously dispersed. The resulting coating liquid having a solids content of 3.03% was maintained at a temperature of 70°C during the stirring and subsequent spraying procedure.

Tablets of Example 5, in an amount of 7000 g, were placed in a 24 inch (approximately 600 mm) coating pan and preheated to a bed temperature of 60°C. The coating liquid was sprayed on to the tablets under the following conditions:

| | |
|---|---|
| outlet air temperature | 48-55°C |
| pan speed | 10-14 rpm, preferably 14 rpm |
| air flow | 300-400 cfm (8.5-11.3 m³/minute) |
| atomizing air pressure | 20-35 psi (138-242 kPa), preferably about 20 psi |
| peristaltic pump setting | 15-40 g/minute/gun (2 gun spray system), preferably 30-40 g/minute/gun |
| tablet bed temp | 37-50°C, preferably about 40°C |

Spraying was continued until an amount of coating solution equivalent to a weight gain of 2.04% had been applied. The resulting coated tablets were cooled to 30°C prior to discharge from the coating pan.

## Claims

1. A pharmaceutical tablet comprising an intraorally disintegratable core and an excipient coating adherent thereto, wherein the coating comprises gellan gum.

2. A pharmaceutical tablet comprising a drug in a therapeutically and/or prophylactically effective amount, the tablet having an intraorally disintegratable core and a coating adherent thereto, wherein the coating comprises gellan gum, and wherein substantially all of the drug is located in the core and is not commingled with gellan gum.

3. The tablet of Claim 2 wherein the drug is selected from the group consisting of ACE inhibitors; α-adrenergic agonists; β-adrenergic agonists; α-adrenergic blockers; β-adrenergic blockers; alcohol deterrents; aldose reductase inhibitors; aldosterone antagonists; amino acids; anabolics; analgesics (both narcotic and non-narcotic); anesthetics; anorexics; antacids; anthelmintics; antiacne agents; antiallergics; antiandrogens; antianginal agents; antianxiety agents; antiarrythmics; antiasthmatics; antibacterial agents and antibiotics; antialopecia and antibaldness agents; antiamebics; antibodies; anticholinergic drugs; anticoagulants and blood thinners; anticolitis drugs; anticonvulsants; anticystitis drugs; antidepressants; antidiabetic agents; antidiarrheals; antidiuretics; antidotes; antiemetics; antiestrogens; antiflatulents; antifungal agents; antigens; antiglaucoma agents; antihistaminics; antihyperactives; antihyperlipoproteinemics; antihypertensives; antihyperthyroid agents; antihypotensives; antihypothyroid agents; anti-infectives; anti-inflammatories (both steroidal and nonsteroidal); antimalarial agents; antimigraine agents; antineoplastics; antiobesity agents; antiparkinsonian agents and antidyskinetics; antipneumonia agents; antiprotozoal agents; antipruritics; antipsoriatics; antipsychotics; antipyretics; antirheumatics; antisecretory agents; anti-shock medications; antispasmodics; antithrombotics; antitumor agents; antitussives; antiulceratives; antiviral agents; anxiolytics; bactericidins; bone densifiers; bronchodilators; calcium channel blockers; carbonic anhydrase inhibitors; cardiotonics and heart stimulants; chemotherapeutics; choleretics; cholinergics; chronic fatigue syndrome medications; CNS stimulants; coagulants; contraceptives; cystic fibrosis medications; decongestants; diuretics; dopamine receptor agonists; dopamine receptor antagonists; enzymes; estrogens; expectorants; gastric hyperactivity medications; glucocorticoids; hemostatics; HMG CoA reductase inhibitors; hormones; hypnotics; immunomodulators; immunosuppressants; laxatives; medicaments for oral and periodontal diseases; miotics; monoamine oxidase inhibitors; mucolytics; multiple sclerosis medications; muscle relaxants; mydriatics; narcotic antagonists; NMDA receptor antagonists; oligonucleotides; ophthalmic drugs; oxytocics; peptides, polypeptides and proteins; polysaccharides; progestogens; prostaglandins; protease inhibitors; respiratory stimulants; sedatives; serotonin uptake inhibitors; sex hormones including androgens; smoking cessation drugs; smooth muscle relaxants; smooth muscle stimulants; thrombolytics; tranquilizers; urinary acidifiers; urinary incontinence medications; vasodilators; vasoprotectants; and combinations thereof

4. The tablet of Claim 2 wherein the drug is a smoking cessation drug.

5. The tablet of Claim 4 wherein the smoking cessation drug is selected from the group consisting ofbupropion, ibogaine, nicotine and metabolites thereof

6. The tablet of Claim 2 wherein the drug is an antibacterial drug.

7. The tablet of Claim 6 wherein the antibacterial drug is an oxazolidinone.

8. The tablet of Claim 7 wherein the oxazolidinone is selected from the group consisting of eperezolid, linezolid, N-[(5*S*)-3-[3-fluoro-4-[4-(2-fluoroethyl)-3-oxo-1-piperazinyl]phenyl-2-oxo-5-oxazolidinyl]methyl]acetamide, (*S*)-N-[[3-[5-(3-pyridyl)thiophen-2-yl]-2-oxo-5-oxazolidinyl]methyl]acetamide, and (*S*)-N-[[3-[5-(4-pyridyl)pyrid-2-yl]-2-oxo-5-oxazolidinyl]methyl]acetamide hydrochloride.

9. The tablet of Claim 2 wherein the drug is an antimigraine agent.

10. The tablet of Claim 9 wherein the antimigraine agent is a 5-HT receptor agonist.

11. The tablet of Claim 10 wherein the 5-HT receptor agonist is selected from the group consisting of almotriptan, eletriptan, frovatriptan, naratriptan, rizatriptan, sumatriptan or zolmitriptan.

12. The tablet of Claim 2 wherein the drug is useful in treating or preventing an ophthalmic disorder.

13. The tablet of Claim 12 wherein the drug is an antiglaucoma or intraocular pressure lowering agent.

14. The tablet of Claim 13 wherein the antiglaucoma or intraocular pressure lowering agent is selected from the group consisting of adrenolone, apraclonidine, brimonidine, dipivefrin, acebutolol, adaprolol, alprenolol, atenolol, betaxolol, bufetolol, bufuralol, bunitrolol, bunolol, bupranolol, carteolol, carvedilol, cetamolol, dexpropanolol, labetalol, levobunolol, metipranolol, metoprolol, nadolol, nifenalol, oxyprenolol, penbutolol, pindolol, practolol, pronethalol, propranolol, sotalol, timolol, tolamolol, toliprolol, vaninolol, acetazolamide, dorzolamide, bimatoprost, latanoprost, travoprost, unoprostone isopropyl and combinations thereof

15. The tablet of Claim 2 wherein the drug is an analgesic, antipyretic or anti-inflammatory agent.

16. The tablet of Claim 15 wherein the analgesic, antipyretic or anti-inflammatory agent is selected from the group consisting of aceclofenac, acemetacin, e-acetamidocaproic acid, acetaminophen, acetaminosalol, acetanilide, acetylsalicylic acid, *S*-adenosylmethionine, alclofenac, alclometasone, alfentanil, algestone, allylprodine, alminoprofen, aloxiprin, alphaprodine, aluminum bis(acetylsalicylate), amcinonide, amfenac, aminochlorthenoxazin, 3-amino-4-hydroxybutyric acid, 2-amino-4-picoline, aminopropylon, aminopyrine, amixetrine, ammonium salicylate, ampiroxicam, amtolmetin guacil, anileridine, antipyrine, antrafenine, apazone, beclomethasone, bendazac, benorylate, benoxaprofen, benzpiperylon, benzydamine, benzylmorphine, bermoprofen, betamethasone, bezitramide, α-bisabolol, bromfenac, *p*-bromoacetanilide, 5-bromosalicylic acid acetate, bromosaligenin, bucetin, bucloxic acid, bucolome, budesonide, bufexamac, bumadizon, buprenorphine, butacetin, butibufen, butophanol, carbamazepine, carbiphene, carprofen, carsalam, celecoxib, chlorobutanol, chloroprednisone, chlorthenoxazin, choline salicylate, cinchophen, cinmetacin, ciramadol, clidanac, clobetasol, clocortolone, clometacin, clonitazene, clonixin, clopirac, cloprednol, clove, codeine, codeine methyl bromide, codeine phosphate, codeine sulfate, cortisone, cortivazol, cropropamide, crotethamide, deflazacort, desomorphine, desonide, desoximetasone, dexamethasone, dexoxadrol, dextromoramide, dezocine, diampromide, diclofenac, difenamizole, difenpiramide, diflorasone, diflucortolone, diflunisal, difluprednate, dihydrocodeine, dihydrocodeinone enol acetate, dihydromorphine, dihydroxyaluminum acetylsalicylate, dimenoxadol, dimepheptanol, dimethylthiambutene, dioxaphetyl butyrate, dipipanone, diprocetyl, dipyrone, ditazol, droxicam, emorfazone, enfenamic acid, enoxolone, epirizole, eptazocine, etersalate, ethenzamide, ethoheptazine, ethoxazene, ethylmethylthiambutene, ethylmorphine, etodolac, etofenamate, etonitazene, etoricoxib, eugenol, felbinac, fenbufen, fenclozic acid, fendosal, fenoprofen, fentanyl, fentiazac, fepradinol, feprazone, floctafenine, fluazacort, flucloronide, flufenamic acid, flumethasone, flunisolide, flunixin, flunoxaprofen, fluocinolone acetonide, fluocinonide, fluocortin butyl, fluocortolone, fluoresone, fluorometholone, fluperolone, flupirtine, fluprednidene, fluprednisolone, fluproquazone, flurandrenolide, flurbiprofen, formocortal, fosfosal, gentisic acid, glafenine, glucametacin, glycol salicylate, guaiazulene, halcinonide, halometasone, haloprednone, hydrocodone, hydrocortamate, hydrocortisone, hydromorphone, hydroxypethidine, ibufenac, ibuprofen, ibuproxam, imidazole salicylate, indomethacin, indoprofen, isofezolac, isoladol, isomethadone, isonixin, isoxepac, isoxicam, ketobemidone, ketoprofen, ketorolac, *p*-lactophenetide, lefetamine, levorphanol, lofentanil, lonazolac, lornoxicam, loxoprofen, lysine acetylsalicylate, mazipredone, meclofenamic acid, medrysone, mefenamic acid, meperidine, meprednisone, meptazinol, mesalamine, metazocine, methadone, methotrimeprazine, methylprednisolone, metiazinic acid, metofoline, metopon, mofebutazone, mofezolac, morazone, morphine, morphine hydrochloride, morphine sulfate, morpholine salicylate, myrophine, nabumetone, nalbuphine, 1-naphthyl salicylate, naproxen, narceine, nefopam, nicomorphine, nifenazone, niflumic acid, nimesulide, 5'-nitro-2'-propoxyacetanilide, norlevorphanol, normethadone, normorphine, norpipanone, olsalazine, opium, oxaceprol, oxametacine, oxaprozin, oxycodone, oxymorphone, oxyphenbutazone, papaveretum, paramethasone, paranyline, parecoxib, parsalmide, pentazocine, perisoxal, phenacetin, phenadoxone, phenazocine, phenazopyridine hydrochloride, phenocoll, phenoperidine, phenopyrazone, phenyl acetylsalicylate, phenylbutazone, phenyl salicylate, phenyramidol, piketoprofen, piminodine, pipebuzone, piperylone, piprofen, pirazolac, piritramide, piroxicam, pranoprofen, prednicarbate, prednisolone, prednisone, prednival, prednylidene, proglumetacin, proheptazine, promedol, propacetamol, propiram, propoxyphene, propyphenazone, proquazone, protizinic acid, proxazole, ramifenazone, remifentanil, rimazolium metilsulfate, rofecoxib, salacetamide, salicin, salicylamide, salicylamide *o*-acetic acid, salicylic acid, salicylsulfuric acid, salsalate, salverine, simetride, sufentanil, sulfasalazine, sulindac, superoxide dismutase, suprofen, suxibuzone, talniflumate, tenidap, tenoxicam, terofenamate, tetrandrine, thiazolinobutazone, tiaprofenic acid, tiaramide, tilidine, tinoridine, tixocortol, tolfenamic acid, tolmetin, tramadol, triamcinolone, tropesin, valdecoxib, viminol, xenbucin, ximoprofen, zaltoprofen and zomepirac.

17. The tablet of Claim 15 wherein the analgesic, antipyretic or anti-inflammatory agent is a selective COX-2 inhibitory drug.

18. The tablet of Claim 17 wherein the selective COX-2 inhibitory drug is a compound having the formula 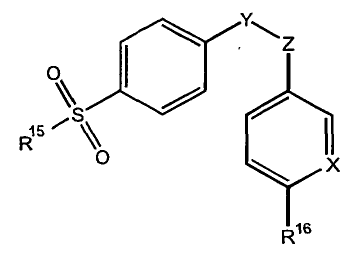 where R¹⁵ is a methyl, amino or imide group, R¹⁶ is hydrogen or a C₁₋₄ alkyl or alkoxy group, X is N or CR¹⁷ where R¹⁷ is hydrogen or halogen, and Y and Z are independently carbon or nitrogen atoms defining adjacent atoms of a five- to six-membered ring that is unsubstituted or substituted at one or more positions with oxo, halo, methyl or halomethyl groups.

19. The tablet of Claim 17 wherein the selective COX-2 inhibitor is selected from the group consisting of celecoxib, deracoxib, valdecoxib, parecoxib, rofecoxib, etoricoxib, 2-(3,5-diffuorophenyl)-3-[4-(methylsulfonyl)phenyl]-2-cyclopenten-1-one, (S)-6,8-dichloro-2-(trifluoromethyl)-2H-1-benzopyran-3-carboxylic acid, 2-(3,4-difluorophenyl)-4-(3-hydroxy-3-methyl-1-butyoxy)-5-[4-(methylsulfonyl)phenyl]-3-(2H)-pyridazinone and salts thereof.

20. The tablet of Claim 2 wherein the drug is an agent useful in treatment and/or prevention of sexual dysfunction.

21. The tablet of Claim 20 wherein the agent is selected from the group consisting of PDE5 inhibitors, cyclic AMP activators, α-adrenergic antagonists and dopaminergic agonists.

22. The tablet of Claim 20 wherein the agent is a compound of formula 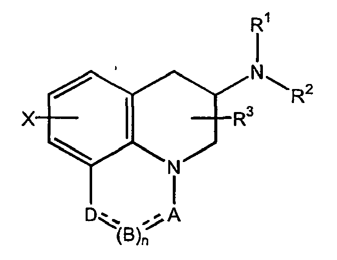 or a pharmaceutically acceptable salt thereof, wherein
R¹, R² and R³ are the same or different and are H, C₁₋₆ alkyl (optionally phenyl substituted), C₃₋₅ alkenyl or alkynyl or C₃₋₁₀ cycloalkyl, or where R³ is as above and R¹ and R² are cyclized with the attached N atom to form pyrrolidinyl, piperidinyl, morpholinyl, 4-methylpiperazinyl or imidazolyl groups;
X is H, F, Cl, Br, I, OH, C₁₋₆ alkyl or alkoxy, CN, carboxamide, carboxyl or (C₁₋₆ alkyl)carbonyl;
A is CH, CH₂, CHF, CHCl, CHBr, CHI, CHCH₃, C=O, C=S, CSCH₃, C=NH, CNH₂, CNHCH₃, CNHCOOCH₃, CNHCN, SO₂ or N;
B is CH, CH₂, CHF, CHCl, CHBr, CHI, C=O, N, NH or NCH₃, and n is 0 or 1; and
D is CH, CH₂, CHF, CHCl, CHBr, CHI, C=O, O, N, NH or NCH₃.

23. The tablet of Claim 20 wherein the agent is a compound of formula 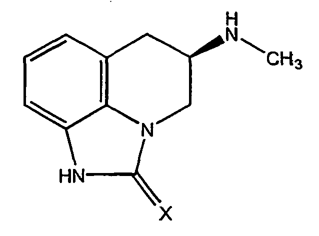 wherein X is O or S, or a pharmaceutically acceptable salt thereof.

24. The tablet of Claim 1 that is suitable for sublingual administration.

25. The tablet of Claim 1 that is suitable for buccal administration.

26. The tablet of Claim 1 wherein the coating is present in an amount representing a weight gain of 0.1% to 5%.

27. The tablet of Claim 1 wherein the gellan gum constitutes 25% to 100% by weight of the coating.

28. The tablet of Claim 1 wherein the gellan gum constitutes 50% to 100% by weight of the coating.

29. The tablet of Claim 1 wherein the coating further comprises at least one additional excipient selected from the group consisting of buffering agents, plasticizers and dispersing and emulsifying agents.

## Patentansprüche

1. Pharmazeutische Tablette, die einen intraoral disintegrierbaren Kern und einen daran haftenden Streckmittelüberzug umfasst, wobei der Überzug Gellangummi umfasst.

2. Pharmazeutische Tablette, die ein Arzneimittel in einer therapeutisch und/oder prophylaktisch wirksamen Menge umfasst, wobei die Tablette einen intraoral disintegrierbaren Kern und einen daran haftenden Überzug aufweist, wobei der Überzug Gellangummi umfasst, und wobei im wesentlichen das gesamte Arzneimittel im Kern lokalisiert ist und mit Gellangummi nicht vermischt ist.

3. Tablette nach Anspruch 2, wobei das Arzneimittel aus der Gruppe von ACE-Inhibitoren, α-Adrenorezeptoragonisten, β-Adrenorezeptoragonisten, α-Adrenorezeptorblockern, β-Adrenorezeptorblockern, Alkoholabschreckungsmitteln, Aldosereduktaseinhibitoren, Aldosteronantagonisten, Aminosäuren, Anabolika, (sowohl narkotischen als auch nichtnarkotischen) Analgetika, Anästhetika, Anorektika, Antazida, Anthelmintika, Antiaknemitteln, Antiallergika, Antiandrogenen, Antianginamitteln, Anxiolytika, Antiarrhythmika, Antasthmatika, antibakteriellen Mitteln und Antibiotika, Antialopeziemitteln und Mitteln gegen Kahlheit, Amöbiziden, Antikörpern, Anticholinergika, Antikoagulantien und Blutverdünnungsmitteln, Antikolitismitteln, Antikonvulsiva, Antizystitismitteln, Antidepressiva, Antidiabetika, Antidiarrhoika, Antidiuretika, Antidoten, Antiemetika, Antiöstrogenen, Antiflatulenzmitteln, Antipilzmitteln, Antigenen, Antiglaukommitteln, Antihistaminika, Antihyperaktiva, Antihyperlipoproteinämika, Antihypertonika, Antihyperthyreosemitteln, Antihypotonika, Antihypothyreosemitteln, Antiinfektiva, (sowohl steroidalen als auch nichtsteroidalen) entzündungshemmenden Mitteln, Antimalariamitteln, Antimigränemitteln, Antineoplastika, Antifettsuchtmitteln, Antiparkinsonmitteln und Antidyskinesiemitteln, Antipneumoniemitteln, Antiprotozoenmitteln, Antipruriginosa, Antipsoriaka, Antipsychotika, Antipyretika, Antirheumatika, antisekretorischen Mitteln, Antischockmedikationen, Spasmolytika, Antithrombotika, Antitumormitteln, Antitussiva, Ulkusmitteln, antiviralen Mitteln, Anxiolytika, Bakterizidinen, die Knochendichte erhöhenden Mitteln, Bronchodilatatoren, Calciumkanalblockern, Carboanhydraseinhibitoren, Kardiotonika und Herzstimulantien, Chemotherapeutika, Choleretika, Cholinergika, Medikationen des chronischen Erschöpfungssyndroms, ZNS-Stimulantien, Koagulantien, Kontrazeptiva, Muskoviszidosemedikationen, Dekongestionsmitteln, Diuretika, Dopaminrezeptoragonisten, Dopaminrezeptorantagonisten, Enzymen, Östrogenen, Expektorantien, Medikationen von Magenhyperaktivität, Glucocorticoiden, Hämostatika, HMG-CoA-Reduktaseinhibitoren, Hormonen, Hypnotika, Immunmodulatoren, Immunsuppressiva, Laxativa, Medikamenten für orale und periodontale Erkrankungen, Miotika, Monoaminoxidaseinhibitoren, Mukolytika, Medikationen von Multipler Skerlose, Muskelrelaxantien, Mydriatika, Narkoseantagonisten, NMDA-Rezeptorantagonisten, Oligonucleotiden, Augenarzneimitteln, Oxytocica, Peptiden, Polypeptiden und Proteinen, Polysacchariden, Progestogenen, Prostaglandinen, Proteaseinhibitoren, Atmungsstimulantien, Sedativa, Serotoninwiederaufnahmehemmern, Sexualhormonen einschließlich Androgenen, Arzneimitteln zum Aufhören von Rauchen, Relaxantien glatter Muskulatur, Stimulantien glatter Muskulatur, Thrombolytika, Tranquilizern, Harnsäuerungsmitteln, Harninkontinenzmedikationen, Vasodilatatoren, Vasoprotektiva und Kombinationen derselben ausgewählt ist.

4. Tablette nach Anspruch 2, wobei das Arzneimittel ein Arzneimittel zum Aufhören von Rauchen ist.

5. Tablette nach Anspruch 4, wobei das Arzneimittel zum Aufhören von Rauchen aus der Gruppe von Bupropion, Ibogain, Nicotin und Metaboliten derselben ausgewählt ist.

6. Tablette nach Anspruch 2, wobei das Arzneimittel ein antibakterielles Arzneimittel ist.

7. Tablette nach Anspruch 6, wobei das antibakterielle Arzneimittel ein Oxazolidinon ist.

8. Tablette nach Anspruch 7, wobei das Oxazolidinon aus der Gruppe von Eperezolid, Linezolid, N-[(5S)-3-[3-Fluor-4-[4-(2-fluorethyl)-3-oxo-1-piperazinyl]phenyl-2-oxo-5-oxazolidinyl]methyl]acetamid, (S)-N-[[3-[5-(3-Pyridyl)thiophen-2-yl]-2-oxo-5-oxazolidinyl]methyl]acetamid und (S)-N-[[3-[5-(4-Pyridyl)pyrid-2-yl]-2-oxo-5-oxazolidinyl]methyl]acetamidhydrochlorid ausgewählt ist.

9. Tablette nach Anspruch 2, wobei das Arzneimittel ein Antimigränemittel ist.

10. Tablette nach Anspruch 9, wobei das Antimigränemittel ein 5-HT-Rezeptoragonist ist.

11. Tablette nach Anspruch 10, wobei der 5-HT-Rezeptoragonist aus der Gruppe von Almotriptan, Eletriptan, Frovatriptan, Naratriptan, Rizatriptan, Sumatriptan oder Zolmitriptan ausgewählt ist.

12. Tablette nach Anspruch 2, wobei das Arzneimittel zur Behandlung oder Prävention einer Augenerkrankung verwendbar ist.

13. Tablette nach Anspruch 12, wobei das Arzneimittel ein Antiglaukommittel oder den intraokulären Druck senkendes Mittel ist.

14. Tablette nach Anspruch 13, wobei das Antiglaukommittel oder den intraokulären Druck senkende Mittel aus der Gruppe von Adrenolon, Apraclonidin, Brimonidin, Dipivefrin, Acebutolol, Adaprolol, Alprenolol, Atenolol, Betaxolol, Bufetolol, Bufuralol, Bunitrolol, Bunolol, Bupranolol, Carteolol, Carvedilol, Cetamolol, Dexpropanolol, Labetalol, Levobunolol, Metipranolol, Metoprolol, Nadolol, Nifenalol, Oxyprenolol, Penbutolol, Pindolol, Practolol, Pronethalol, Propranolol, Sotalol, Timolol, Tolamolol, Toliprolol, Vaninolol, Acetazolamid, Dorzolamid, Bimatoprost, Latanoprost, Travoprost, Unoprostonisopropyl und Kombinationen derselben ausgewählt ist.

15. Tablette nach Anspruch 2, wobei das Arzneimittel ein Analgetikum, Antipyretikum oder entzündungshemmendes Mittel ist.

16. Tablette nach Anspruch 15, wobei das Analgetikum, Antipyretikum oder entzündungshemmende Mittel aus der Gruppe von Aceclofenac, Acemetacin, e-Acetamidocapronsäure, Acetaminophen, Acetaminosalol, Acetanilid, Acetylsalicylsäure, S-Adenosylmethionin, Alclofenac, Alclometason, Alfentanil, Algeston, Allylprodin, Alminoprofen, Aloxiprin, Alphaprodin, Aluminiumbis(acetylsalicylat), Amcinonid, Amfenac, Aminochlorthenoxazin, 3-Amino-4-hydroxybuttersäure, 2-Amino-4-picolin, Aminopropylon, Aminopyrin, Amixetrin, Ammoniumsalicylat, Ampiroxicam, Amtolmetinguacil, Anileridin, Antipyrin, Antrafenin, Apazon, Beclomethason, Bendazac, Benorylat, Benoxaprofen, Benzpiperylon, Benzydamin, Benzylmorphin, Bermoprofen, Betamethason, Bezitramid, α-Bisabolol, Bromfenac, p-Bromacetanilid, 5-Bromsalicylsäureacetat, Bromsaligenin, Bucetin, Bucloxinsäure, Bucolom, Budesonid, Bufexamac, Bumadizon, Buprenorphin, Butacetin, Butibufen, Butophanol, Carbamazepin, Carbiphen, Carprofen, Carsalam, Celecoxib, Chlorbutanol, Chlorprednison, Chlorthenoxazin, Cholinsalicylat, Cinchophen, Cinmetacin, Ciramadol, Clidanac, Clobetasol, Clocortolon, Clometacin, Clonitazen, Clonixin, Clopirac, Cloprednol, Clove, Codein, Codeinmethylbromid, Codeinphosphat, Codeinsulfat, Cortison, Cortivazol, Cropropamid, Crotethamid, Deflazacort, Desomorphin, Desonid, Desoximetason, Dexamethason, Dexoxadrol, Dextromoramid, Dezocin, Diampromid, Diclofenac, Difenamizol, Difenpiramid, Diflorason, Diflucortolon, Diflunisal, Difluprednat, Dihydrocodein, Dihydrocodeinenolacetat, Dihydromorphin, Dihydroxyaluminiumacetylsalicylat, Dimenoxadol, Dimepheptanol, Dimethylthiambuten, Dioxaphetylbutyrat, Dipipanon, Diprocetyl, Dipyron, Ditazol, Droxicam, Emorfazon, Enfenamsäure, Enoxolon, Epirizol, Eptazocin, Etersalat, Ethenzamid, Ethoheptazin, Ethoxazen, Ethylmethylthiambuten, Ethylmorphin, Etodolac, Etofenamat, Etonitazen, Etoricoxib, Eugenol, Felbinac, Fenbufen, Fenclozinsäure, Fendosal, Fenoprofen, Fentanyl, Fentiazac, Fepradinol, Feprazon, Floctafenin, Fluazacort, Flucloronid, Flufenamsäure, Flumethason, Flunisolid, Flunixin, Flunoxaprofen, Fluocinolonacetonid, Fluocinonid, Fluocortinbutyl, Fluocortolon, Fluoreson, Fluormetholon, Fluperolon, Flupirtin, Flupredniden, Fluprednisolon, Fluproquazon, Flurandrenolid, Flurbiprofen, Formocortal, Fosfosal, Gentisinsäure, Glafenin, Glucametacin, Glykolsalicylat, Guaiazulen, Halcinonid, Halometason, Haloprednon, Hydrocodon, Hydrocortamat, Hydrocortison, Hydromorphon, Hydroxypethidin, Ibufenac, Ibuprofen, Ibuproxam, Imidazolsalicylat, Indomethacin, Indoprofen, Isofezolac, Isoladol, Isomethadon, Isonixin, Isoxepac, Isoxicam, Ketobemidon, Ketoprofen, Ketorolac, p-Lactophenetid, Lefetamid, Levorphanol, Lofentanil, Lonazolac, Lornoxicam, Loxoprofen, Lysinacetylsalicylat, Mazipredon, Meclofenamsäure, Medryson, Mefenamsäure, Meperidin, Meprednison, Meptazinol, Mesalamin, Metazocin, Methadon, Methotrimeprazin, Methylprednisolon, Metiazinsäure, Metofolin, Metopon, Mofebutazon, Mofezolac, Morazon, Morphin, Morphinhydrochlorid, Morphinsulfat, Morpholinsalicylat, Myrophin, Nabumeton, Nalbuphin, 1-Naphthylsalicylat, Naproxen, Narcein, Nefopam, Nicomorphin, Nifenazon, Nifluminsäure, Nimesulid, 5'-Nitro-2'-propoxyacetanilid, Norlevorphanol, Normethadon, Normorphin, Norpipanon, Olsalazin, Opium, Oxaceprol, Oxametacin, Oxaprozin, Oxycodon, Oxymorphon, Oxyphenbutazon, Papaveretum, Paramethason, Paranylin, Parecoxib, Parsalmid, Pentazocin, Perisoxal, Phenacetin, Phenadoxon, Phenazocin, Phenazopyridinhydrochlorid, Phenocoll, Phenoperidin, Phenopyrazon, Phenylacetylsalicylat, Phenylbutazon, Phenylsalicylat, Phenyramidol, Piketoprofen, Piminodin, Pipebuzon, Piperylon, Piprofen, Pirazolac, Piritramid, Piroxicam, Pranoprofen, Prednicarbat, Prednisolon, Prednison, Prednival, Prednyliden, Proglumetacin, Propheptazin, Promedol, Propacetamol, Propiram, Propoxyphen, Propyphenazon, Proquazon, Protizinsäure, Proxazol, Ramifenazon, Remifentanil, Rimazoliummetilsulfat, Rofecoxib, Salacetamid, Salicin, Salicylamid, Salicylamid-o-essigsäure, Salicylsäure, Salicylschwefelsäure, Salsalat, Salverin, Simetrid, Sufentanil, Sulfasalazin, Sulindac, Superoxiddismutase, Suprofen, Suxibuzon, Talniflumat, Tenidap, Tenoxicam, Terofenamat, Tetrandrin, Thiazolinobutazon, Tiaprofensäure, Tiaramid, Tilidin, Tinoridin, Tixocortol, Tolfenamsäure, Tolmetin, Tramadol, Triamcinolon, Tropesin, Valdecoxib, Viminol, Xenbucin, Ximoprofen, Zaltoprofen und Zomepirac ausgewählt ist.

17. Tablette nach Anspruch 15, wobei das Analgetikum, Antipyretikum oder entzündungshemmende Mittel ein selektives COX-2 hemmendes Arzneimittel ist.

18. Tablette nach Anspruch 17, wobei das selektive, COX-2 hemmende Arzneimittel eine Verbindung der Formel 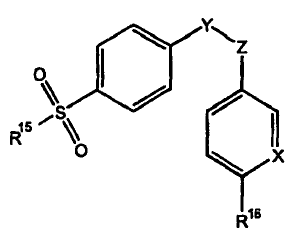 ist, worin R¹⁵ für eine Methyl-, Amino- oder Imidgruppe steht, R¹⁶ für Wasserstoff oder eine C₁-C₄-Alkyl- oder Alkoxygruppe steht, X für N oder CR¹⁷ steht, worin R¹⁷ Wasserstoff oder Halogen ist, und Y und Z unabhängig voneinander Kohlenstoff- oder Stickstoffatome sind, die angrenzende Atome eines 5- bis 6-gliedrigen Rings festlegen, der an einer oder mehreren Positionen mit Oxo-, Halogen-, Methyl- oder Halogenmethylgruppen substituiert ist.

19. Tablette nach Anspruch 17, wobei der selektive COX-2-Inhibitor aus der Gruppe von Celecoxib, Deracoxib, Valdecoxib, Parecoxib, Rofecoxib, Etoricoxib, 2-(3,5-Difluorphenyl)-3-[4-(methylsulfonyl)phenyl]-2-cyclopenten-1-on, (S)-6,8-Dichlor-2-(trifluormethyl)-2H-1-benzopyran-3-carbonsäure, 2-(3,4-Difluorphenyl)-4-(3-hydroxy-3-methyl-1-butoxy)-5-[4-(methylsulfonyl)phenyl]-3-(2H)-pyridazinon und Salzen derselben ausgewählt ist.

20. Tablette nach Anspruch 2, wobei das Arzneimittel ein bei der Behandlung und/oder Prävention von sexueller Dysfunktion verwendbares Mittel ist.

21. Tablette nach Anspruch 20, wobei das Mittel aus der Gruppe von PDE5-Inhibitoren, cyclischen AMP-Aktivatoren, α-Adrenorezeptorantagonisten und dopaminergen Agonisten ausgewählt ist.

22. Tablette nach Anspruch 20, wobei das Mittel eine Verbindung der Formel 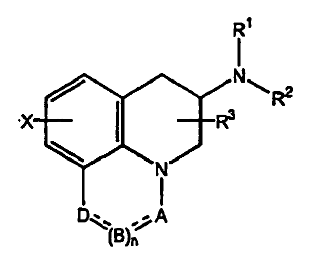 oder ein pharmazeutisch akzeptables Salz derselben ist, worin
R¹, R² und R³ gleich oder verschieden sind und für H, C₁-C₆-Alkyl (optional phenylsubstituiert), C₃-C₅-Alkenyl oder -Alkinyl oder C₃-C₁₀-Cycloalkyl stehen, oder worin R³ wie oben angegeben ist und R¹ und R² mit dem daran gebundenen N-Atom unter Bildung von Pyrrolidinyl-, Piperidinyl-, Morpholinyl-, 4-Methylpiperazinyl- oder Imidazolylgruppen cyclisiert sind;
X für H, F, Cl, Br, I, OH, C₁-C₆-Alkyl oder Alkoxy, CN, Carboxamid, Carboxyl oder (C₁-C₆-Alkyl)carbonyl steht; A für CH, CH₂, CHF, CHCl, CHBr, CHI, CHCH₃, C=O, C=S, CSCH₃, C=NH, CNH₂, CNHCH₃, CNHCOOCH₃, CNHCN, SO₂ oder N steht;
B für CH, CH₂, CHF, CHCl, CHBr, CHI, C=O, N, NH oder NCH₃ steht und n für 0 oder 1 steht; und
D für CH, CH₂, CHF, CHCl, CHBr, CHI, C=O, O, N, NH oder NCH₃ steht.

23. Tablette nach Anspruch 20, wobei das Mittel eine Verbindung der Formel 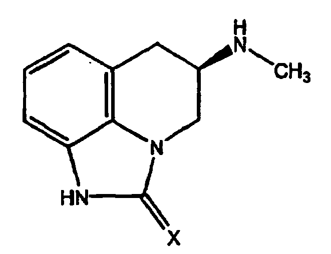 worin X für O oder S steht, oder ein pharmazeutisch akzeptables Salz derselben ist.

24. Tablette nach Anspruch 1, die zur sublingualen Verabreichung geeignet ist.

25. Tablette nach Anspruch 1, die zur bukkalen Verabreichung geeignet ist.

26. Tablette nach Anspruch 1, wobei der Überzug in einer Menge vorhanden ist, die eine Gewichtszunahme von 0,1 % bis 5 % darstellt.

27. Tablette nach Anspruch 1, wobei der Gellangummi 25 bis 100 Gew.-% des Überzugs ausmacht.

28. Tablette nach Anspruch 1, wobei der Gellangummi 50 bis 100 Gew.-% des Überzugs ausmacht.

29. Tablette nach Anspruch 1, wobei der Überzug ferner mindestens ein weiteres Streckmittel umfasst, das aus der Gruppe von Puffermitteln, Weichmachern und Dispergiermitteln und Emulgatoren ausgewählt ist.

## Revendications

1. Comprimé pharmaceutique comprenant un noyau apte au délitement intra-oral et un enrobage d'excipient adhérant à celui-ci, dans lequel l'enrobage comprend de la gomme gellane.

2. Comprimé pharmaceutique comprenant un médicament en une quantité thérapeutiquement et/ou prophylactiquement efficace, le comprimé comprenant un noyau apte au délitement intra-oral et un enrobage adhérant à ce noyau, dans lequel l'enrobage comprend la gomme gellane, et dans lequel pratiquement la totalité du médicament est située dans le noyau et n'est pas mêlée à la gomme gellane.

3. Comprimé suivant la revendication 2, dans lequel le médicament est choisi dans le groupe consistant en des inhibiteurs de ACE ; des agonistes α-adrénergiques ; des agonistes β-adrénergiques ; des agents de blocage α-adrénergique ; des agents de blocage β-adrénergique ; des agents dissuadant la consommation d'alcool ; des inhibiteurs d'aldose-réductase ; des antagonistes d'aldostérone ; des aminoacides ; des anabolisants ; des analgésiques (narcotiques et non narcotiques) ; des anesthésiques ; des anorexigènes ; des anti-acides ; des anthelmintiques ; des agents antiacnéiques ; des antiallergiques ; des antiandrogènes ; des agents antiangineux ; des anxiolytiques ; des antiarythmiques ; des antiasthmatiques ; des agents antibactériens et des antibiotiques ; des agents contre l'alopécie et contre la calvitie ; des antiamibiens ; des anticorps ; des médicaments anticholinergiques ; des anticoagulants et des fluidifiants sanguins ; des médicaments contre la colite ; des anticonvulsivants ; des médicaments anticystite ; des antidépresseurs ; des agents antidiabétiques ; des antidiarrhéiques ; des antidiurétiques ; des antidotes ; des antiémétiques ; des antioestrogènes ; des antiflatulents ; des agents antifongiques ; des antigènes ; des agents antiglaucomes ; des antihistaminiques ; des antihyperactifs ; des antihyperlipoprotéinémiques ; des antihypertensifs ; des agents antihyperthyroïdiens ; des antihypotensifs ; des agents antihypothyroïdiens ; des anti-infectieux ; des anti-inflammatoires (stéroïdiens et non stéroïdiens) ; des agents antipaludiques ; des antimigraineux ; des antinéoplasiques ; des agents contre l'obésité ; des antiparkinsoniens ; des antidyskinésiques ; des agents contre la pneumonie ; des agents contre les protozoaires ; des antipruritiques ; des antipsoriasiques ; des antipsychotiques ; des antipyrétiques ; des antirhumatismaux ; des agents antisécrétoires ; des médicaments contre l'état de choc ; des antispasmodiques ; des antithrombotiques ; des agents antitumoraux ; des antitussifs ; des antiulcératifs ; des agents antiviraux ; des anxiolytiques ; des bactéricidines ; des agents de densification osseuse ; des bronchodilatateurs ; des agents de blocage du canal calcium ; des inhibiteurs d'anhydrase carbonique ; des cardiotoniques et des stimulants cardiaques ; des agents chimiothérapeutiques ; des cholérétiques ; des cholinergiques ; des médicaments contre le syndrome de fatigue chronique ; des stimulants du SNC ; des coagulants ; des contraceptifs ; des médicaments contre la fibrose kystique ; des décongestionnants ; des diurétiques ; des agonistes du récepteur de dopamine ; des antagonistes du récepteur de dopamine ; des enzymes ; des oestrogènes ; des expectorants ; des médicaments contre l'hyperactivité gastrique ; des glucocorticoïdes ; des hémostatiques ; des inhibiteurs de HMG-CoA-réductase ; des hormones ; des hypnotiques ; des immunomodulateurs ; des immunosuppresseurs ; des laxatifs ; des médicaments pour des maladies orales et périodontiques ; des miotiques ; des inhibiteurs de monoamine-oxydase ; des mucolytiques ; des médicaments contre la sclérose en plaques ; des myorelaxants ; des mydriatiques ; des antagonistes des narcotiques ; des antagonistes des récepteurs de NMDA ; des oligonucléotides ; des médicaments ophtalmiques ; des ocytociques ; des peptides, des polypeptides et des protéines ; des polysaccharides ; des hormones progestatives ; des prostaglandines ; des inhibiteurs de protéases ; des stimulants respiratoires ; des sédatifs ; des inhibiteurs d'absorption de sérotonine ; des hormones sexuelles comprenant des androgènes ; des médicaments pour cesser de fumer ; des relaxants des muscles lisses ; des stimulants des muscles lisses ; des thrombolytiques ; des tranquillisants ; des acidifiants urinaires ; des médicaments contre l'incontinence urinaire ; des vasodilatateurs ; des vasoprotecteurs ; et leurs associations.

4. Comprimé suivant la revendication 2, dans lequel le médicament est un médicament pour cesser de fumer.

5. Comprimé suivant la revendication 4, dans lequel le médicament pour cesser de fumer est choisi dans le groupe consistant en bupropion, ibogaïne, nicotine et ses métabolites.

6. Comprimé suivant la revendication 2, dans lequel le médicament est un médicament antibactérien.

7. Comprimé suivant la revendication 6, dans lequel le médicament antibactérien est une oxazolidinone.

8. Comprimé suivant la revendication 7, dans lequel l'oxazolidinone est choisi dans le groupe consistant en épérézolide, linézolide, N-[(5*S*)-3-[3-fluoro-4-[4-(2-fluoréthyl)-3-oxo-1-pipérazinyl]phényl-2-oxo-5-oxazolidinyl]méthyl]acétamide, (*S*)-N-[[3-[5-(3-pyridyl)thiophène-2-yl]-2-oxo-5-oxazolidinyl]méthyl]acétamide et chlorhydrate de (*S*)-N-[[3-[5-(4-pyridyl)pyrid-2-yl]-2-oxo-5-oxazolidinyl]méthyl]acétamide.

9. Comprimé suivant la revendication 2, dans lequel le médicament est un agent antimigraineux.

10. Comprimé suivant la revendication 9, dans lequel l'agent migraineux est un agoniste du récepteur de 5-HT.

11. Comprimé suivant la revendication 10, dans lequel l'agoniste du récepteur de 5-HT est choisi dans le groupe consistant en almotriptan, élétriptan, frovatriptan, naratriptan, rizatriptan, sumatriptan et zolmitriptan.

12. Comprimé suivant la revendication 2, dans lequel le médicament est utile dans le traitement ou la prévention d'un trouble ophtalmique.

13. Comprimé suivant la revendication 12, dans lequel le médicament est un agent contre le glaucome ou un agent abaissant la pression intraoculaire.

14. Comprimé suivant la revendication 13, dans lequel l'agent contre le glaucome ou agent abaissant la pression intraoculaire est choisi dans le groupe consistant en adrénolone, apraclonidine, brimonidine, dipivéfrin, acébutolol, adaprolol, alprénolol, aténolol, bétaxolol, bufétolol, bufuralol, bunitrolol, bunolol, bupranolol, cartéolol, carvédilol, cétamolol, dexpropanolol, labétalol, lévobunolol, métipranolol, métoprolol, nadolol, nifénalol, oxyprénolol, penbutolol, pindolol, practolol, pronéthalol, propranolol, sotalol, timolol, tolamolol, toliprolol, vaninolol, acétazolamide, dorzolamide, bimatoprost, latanoprost, travoprost, unoprostone-isopropyl et leurs associations.

15. Comprimé suivant la revendication 2, dans lequel le médicament est un agent analgésique, antipyrétique ou anti-inflammatoire.

16. Comprimé suivant la revendication 15, dans lequel l'agent analgésique, antipyrétique ou anti-inflammatoire est choisi dans le groupe consistant en acéclofénac, acémétacine, acide ε-acétamidocaproïque, acétaminophène, acétaminosalol, acétanilide, acide acétylsalicylique, *S-*adénosylméthionine, alclofénac, alclométasone, alfentanil, algestone, allylprodine, alminoprofène, aloxiprine, alphaprodine, bis(acétylsalicylate) d'aluminium, amcinonide, amfénac, aminochlorthénoxazine, acide 3-amino-4-hydroxybutyrique, 2-amino-4-picoline, aminopropylon, aminopyrine, amixétrine, salicylate d'ammonium, ampiroxicam, amtolmétine-guacile, aniléridine, antipyrine, antrafénine, apazone, béclométhasone, bendazac, bénorylate, bénoxaprofène, benzpipérylon, benzydamine, benzylmorphine, bermoprofène, bétaméthasone, bézitramide, α-bisabolol, bromfénac, *p*-bromoacétanilide, acétate d'acide 5-bromosalicylique, bromosaligénine, bucétine, acide bucloxique, bucolome, budésonide, bufexamac, bumadizon, buprénorphine, butacétine, butibufène, butophanol, carbamazépine, carbiphène, carprofène, carsalam, célécoxib, chlorobutanol, chloroprednisone, chlorthénoxazine, salicylate de choline, cinchophène, cinmétacine, ciramadol, clidanac, clobétasol, clocortolone, clométacine, clonitazène, clonixine, clopirac, cloprednol, essence de girofle, codéine, méthylbromure de codéine, phosphate de codéine, sulfate de codéine, cortisone, cortivazol, cropropamide, crotéthamide, déflazacort, désomorphine, désonide, désoximétasone, dexaméthasone, dexoxadrol, dextromoramide, dézocine, diampromide, diclofénac, difénamizole, difénamizole, difenpiramide, diflorasone, diflucortolone, diflunisal, difluprednate, dihydrocodéine, acétate d'énol de dihydrocodéinone, dihydromorphine, acétylsalicylate de dihydroxyaluminium, diménoxadol, dimépheptanol, diméthylthiambutène, butyrate de dioxaphétyl, dipipanone, diprocétyle, dipyrone, ditazol, droxicam, émorfazone, acide enfénamique, énoxolone, épirizole, eptazocine, étersalate, éthenzamide, éthoheptazine, éthoxazène, éthylméthylthiambutène, éthylmorphine, étodolac, étofénamate, étonitazène, étoricoxib, eugénol, felbinac, fenbufène, acide fenclozique, fendosal, fénoprofène, fentanyl, fentiazac, fépradinol, féprazone, floctafénine, fluazacort, flucloronide, acide flufénamique, fluméthasone, flunisolide, flunixine, flunoxaprofène, acétonide de fluocinolone, fluocinonide, fluocortine-butyl, fluocortolone, fluorésone, fluorométholone, flupérolone, flupirtine, fluprednidène, fluprednisolone, fluproquazone, flurandrénolide, flurbiprofène, formocortal, fosfosal, acide gentisique, glafénine, glucamétacine, salicylate de glycol, guaïazulène, halcinonide, halométasone, haloprednone, hydrocodone, hydrocortamate, hydrocortisone, hydromorphone, hydroxypéthidine, ibufénac, ibuprofène, ibuproxam, salicylate d'imidazole, indométhacine, indoprofène, isofézolac, isoladol, isométhadone, isonixine, isoxépac, isoxicam, kétobémidone, kétoprofène, kétorolac, *p*-lactophénétide, léfétamine, lévorphanol, lofentanil, lonazolac, lornoxicam, loxoprofène, acétylsalicylate de lysine, maziprédone, acide méclofénamique, médrysone, acide méfénamique, mépéridine, méprednisone, meptazinol, mésalamine, métazocine, méthadone, méthotriméprazine, méthylprednisolone, acide métiazinique, métofoline, métopon, mofébutazone, mofézolac, morazone, morphine, chlorhydrate de morphine, sulfate de morphine, salicylate de morphine, myrophine, nabumétone, nalbuphine, salicylate de 1-naphtyle, naproxène, narcéine, néfopam, nicomorphine, nifénazone, acide niflumique, nimésulide, 5'-nitro-2'-propoxyacétanilide, norlévorphanol, norméthadone, normorphine, norpipanone, olsalazine, opium, oxacéprol, oxamétacine, oxaprozine, oxycodone, oxymorphone, oxyphenbutazone, papavérétum, paraméthasone, paranyline, parécoxib, parsalmide, pentazocine, périsoxal, phénacétine, phénadoxone, phénazocine, chlorhydrate de phénazopyridine, phénocoll, phénopéridine, phénopyrazone, acétylsalicylate de phényle, phénylbutazone, salicylate de phényle, phényramidol, pikétoprofène, piminodine, pipébuzone, pipérylone, piprofène, pirazolac, piritramide, piroxicam, pranoprofène, prednicarbate, prednisolone, prednisone, prednival, prednylidène, proglumétacine, proheptazine, promédol, propacétamol, propiram, propoxyphène, propyphénazone, proquazone, acide protizinique, proxazole, ramifénazone, rémifentanil, méthylsulfate de rimazolium, rofécoxib, salacétamide, salicine, salicylamide, acide salicylamide-*O*-acétique, acide salicylique, acide salicylsulfurique, salsalate, salvérine, simétride, sufentanil, sulfasalazine, sulindac, superoxyde-dismutase, suprofène, suxibuzone, talniflumate, ténidap, ténoxicam, térofénamate, tétrandrine, thiazolinobutazone, acide tiaprofénique, tiaramide, tilidine, tinoridine, tixocortol, acide tolfénamique, tolmétine, tramadol, triamcinolone, tropésine, valdécoxib, viminol, xenbucine, ximoprofène, zaltoprofène et zomépirac.

17. Comprimé suivant la revendication 15, dans lequel l'agent analgésique, antipyrétique ou anti-inflammatoire est un médicament inhibiteur de COX-2 sélectif.

18. Comprimé suivant la revendication 17, dans lequel le médicament inhibiteur de COX-2 sélectif est un composé répondant à la formule 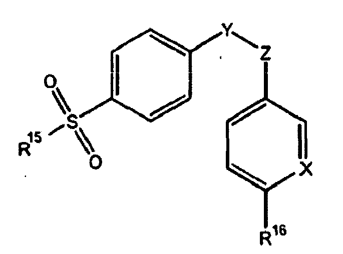 dans laquelle R¹⁵ représente un groupe méthyle, amino ou imide, R¹⁶ représente un atome d'hydrogène ou un groupe alkyle en C₁ à C₄ ou hydroxy, X représente un atome de N ou un groupe CR¹⁷ dans lequel R¹⁷ représente un atome d'hydrogène ou d'halogène, et Y et Z représentent indépendamment des atomes de carbone ou d'azote définissant des atomes adjacents d'un noyau penta- ou hexagonal qui est non substitué ou substitué à une ou plusieurs positions avec des groupes oxo, halogéno, méthyle ou halogénométhyle.

19. Comprimé suivant la revendication 17, dans lequel l'inhibiteur de COX-2 sélectif est choisi dans le groupe consistant en célécoxib, déracoxib, valdécoxib, parécoxib, rofécoxib, étoricoxib, 2-(3,5-difluorophényl)-3-[4-(méthylsulfonyl)phényl] -2-cyclopentène-1-one, acide (S) -6, 8-dichloro-2-(trifluorométhyl)-2H-1-benzopyranne-3-carboxylique, 2-(3,4-difluorophényl)-4-(3-hydroxy-3-méthyl-1-butoxy)-5-[4-(méthylsulfonyl)phényl]-3-(2H)-pyridazinone et leurs sels.

20. Comprimé suivant la revendication 2, dans lequel le médicament est un agent utile dans le traitement et/ou la prévention d'un dysfonctionnement sexuel.

21. Comprimé suivant la revendication 20, dans lequel l'agent est choisi dans le groupe consistant en inhibiteurs de PDE5, activateurs de AMP cyclique, antagonistes α-adrénergiques et agonistes dopaminergiques.

22. Comprimé suivant la revendication 20, dans lequel l'agent est un composé de formule 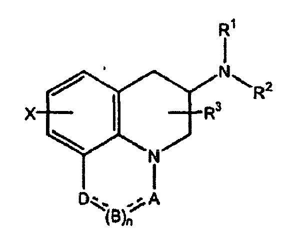 ou un de ses sels pharmaceutiquement acceptables, formule dans laquelle
R¹, R² et R³ sont identiques ou différents et représentent H, des groupes alkyle en C₁ à C₆ (facultativement à substituant phényle), alcényle ou alcynyle en C₃ à C₅ ou cycloalkyle en C₃ à C₁₀, ou bien R³ répond à la définition précitée et R¹ et R² sont cyclisés avec l'atome de N fixé pour former des groupes pyrrolidinyle, pipéridinyle, morpholinyle, 4-méthylpipérazinyle ou imidazolyle ;
X représente H, F, Cl, Br, I, un groupe OH, alkyle ou alkoxy en C₁ à C₆, CN, carboxamide, carboxyle ou (alkyle en C₁ à C₆)carbonyle ;
A représente un groupe CH, CH₂, CHF, CHCl, CHBr, CHI, CHCH₃, C=O, C=S, CSCH₃, C=NH, CNH₂, CNHCH₃, CNHCOOCH₃, CNHCN, SO₂ ou N ;
B représente un groupe CH, CH₂, CHF, CHCl, CHBr, CHI, C=O, N, NH ou NCH₃, et n est égal à 0 ou 1 ; et
D représente un groupe CH, CH₂, CHF, CHCl, CHBr, CHI, C=O, O, N, NH ou NCH₃.

23. Comprimé suivant la revendication 20, dans lequel l'agent est un composé de formule 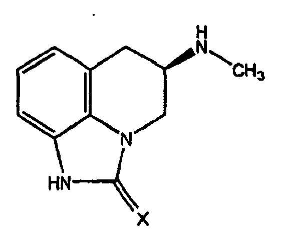 dans laquelle X représente O ou S, ou un de ses sels pharmaceutiquement acceptables.

24. Comprimé suivant la revendication 1, qui est apte à l'administration sublinguale.

25. Comprimé suivant la revendication 1, qui est apte à l'administration buccale.

26. Comprimé suivant la revendication 1, dans lequel l'enrobage est présent en une quantité représentant un gain de poids de 0,1 % à 5 %.

27. Comprimé suivant la revendication 1, dans lequel la gomme gellane représente 25 % à 100 % en poids de l'enrobage.

28. Comprimé suivant la revendication 1, dans lequel la gomme gellane représente 50 % à 100 % en poids de l'enrobage.

29. Comprimé suivant la revendication 1, dans lequel l'enrobage comprend en outre au moins un excipient supplémentaire choisi dans le groupe consistant en des tampons, des plastifiants et des agents dispersants et émulsionnants.
